# EUROPEAN PATENT APPLICATION

(11) **EP 4 129 155 A1**
(43) Date of publication of application: **08.02.2023**
(21) Application number: 21781620.6
(22) Date of filing: 25.03.2021
(51) Int. Cl.: A61B 3/028, A61B 5/16

(54) **OPTOMETRY SYSTEM AND OPTOMETRY PROGRAM**

(30) Priority: 31.03.2020 JP 2020063526; 01.12.2020 JP 2020199931
(71) Applicant: Nidek Co., Ltd., Gamagori-shi, Aichi, 443-0038 (JP)
(72) Inventor: SHIMAZAKI Arisa, Gamagori-shi Aichi 443-0038 (JP); HORINO Taeko, Gamagori-shi Aichi 443-0038 (JP); HIRAYAMA Yukito, Gamagori-shi Aichi 443-0038 (JP); TAKII Michihiro, Gamagori-shi Aichi 443-0038 (JP); SEKO Jouji, Gamagori-shi Aichi 443-0038 (JP)
(74) Representative: Hoefer & Partner Patentanwälte mbB
(86) International application number: PCT/JP2021/012742
(87) International publication number: WO 2021/200604

(57) **Abstract**

This optometry system, for presenting a test chart to an eye being tested and subjectively measuring an optical characteristic of the eye being tested, is provided with a setting means which sets a reference value based on the subject's reaction time to a test chart, a reaction input means which inputs the response of the subject reading the test chart, a control means which automatically advances the test on the basis of an input signal from the response input means, and a guidance information output means which, on the basis of the reference value set by the setting means, outputs guidance information for guiding the subject to input a response during testing of the eye being tested. Hereby, it is possible to accurately carry out a subjective test on the eye being tested.

## Description

### TECHNICAL FIELD

The present disclosure relates to an optometry system and an optometry program for subjectively measuring an optical characteristic of a subject eye.

### BACKGROUND ART

As an optometry system, a subjective optometry device that subjectively measures an optical characteristic of a subject eye is used. For example, a subjective optometry device measures an optical characteristic of a subject eye by arranging optical members in front of the subject eye and presenting a visual target to the subject eye via the optical member (refer to Patent Literature 1).

### CITATION LIST

### PATENT LITERATURE

Patent Literature 1: JPH05-176893A

### SUMMARY OF INVENTION

Recently, for reasons such as the desire to efficiently examine the eyes of many examinees, there has been a demand for a mechanism for easily performing a subjective examination of the subject eye. Therefore, the inventors have studied so-called self-optometry in which an examinee proceeds with the optometry by himself or herself. A first aspect is that it may not be possible to accurately obtain examination results in some cases since examinee are in an environment where it is difficult to communicate with examiners due to self-optometry.

Further, when measuring the optical characteristic of the subject eye, the examiner proceeds with the measurement according to the answers of the examinee and obtains the measurement results. For example, in a refractive correction examination, the examiner changes the correction power for correcting the subject eye, and based on the answers of the examinee each time, determines whether or not the visual target can be seen in the subject eye. However, the examinee may give a correct answer by chance. A second aspect is that, in such a case, it is difficult for the examiner to understand whether or not the subject eye can actually see the visual target, and there is a possibility that the correct measurement result of the subject eye cannot be obtained.

In view of the above aspects, an object of the present disclosure is to provide at least one of an optometry system and an optometry program capable of accurately performing a subjective examination, or an optometry system and an optometry program capable of accurately measuring the optical characteristic of a subject eye.

According to a first aspect of the present disclosure, there is provided an optometry system for subjectively measuring an optical characteristic of a subject eye by presenting an examination visual target to the subject eye, the optometry system including: a setting means that sets a reference value based on a reaction time of an examinee with respect to the examination visual target; a response input means by which the examinee reading the examination visual target inputs an answer; a control means that automatically proceeds with an examination, based on an input signal from the response input means; and a leading information output means that outputs leading information, during the examination of the subject eye, for leading the examinee to input the answer, based on the reference value set by the setting means.

In the optometry system according to the first aspect of the present disclosure, there is provided an optometry program used in an optometry system for subjectively measuring an optical characteristic of a subject eye by presenting an examination visual target to the subject eye, the optometry program being executed by a processor to cause the optometry system to perform: a setting step of setting a reference value based on a reaction time of an examinee with respect to the examination visual target; an answering step in which the examinee reading the examination visual target inputs an answer; a control step of automatically proceeding with an examination, based on an input signal from the response input means; and a leading information output step of outputting leading information, during the examination of the subject eye, for leading the examinee to input the answer, based on the reference value set by the setting means.

According to a second aspect of the present disclosure, there is provided an optometry system for subjectively measuring an optical characteristic of a subject eye, the optometry system including: a visual target presenting means that emits a target light flux toward the subject eye; a correction means that changes an optical characteristic of the target light flux; a control means that controls an operation of the optometry system; a reaction time acquisition means that acquires a reaction time required from start of measurement to a response, based on a start timing at which the measurement of the subject eye is started, which is a start timing at which the visual target presenting means or the correction means is controlled by the control means, and a response timing after the start timing, which is a response timing at which a response signal for the measurement is input by the response input means; and an output means that outputs the reaction time acquired by the reaction time acquisition means.

In the optometry system according to the second aspect of the present disclosure, there is provided an optometry program used in an optometry system for subj ectively measuring an optical characteristic of a subject eye, the optometry system including a visual target presenting means that emits a target light flux toward the subject eye, a correction means that changes an optical characteristic of the target light flux, and a control means that controls an operation of the optometry system, the control means being capable of controlling at least one of the visual target presenting means or the correcting means, the optometry program being executed by the control means to cause the optometry system to perform: a reaction time acquisition step of acquiring a reaction time required from start of measurement to response based on a start timing at which the measurement of the subject eye is started, which is a start timing at which the visual target presenting means or the correction means is controlled by the control means, and a response timing after the start of the measurement, which is a response timing at which a response signal for the measurement is input by response input means, and an output step of outputting the reaction time acquired by the reaction time acquisition means.

### BRIEF DESCRIPTION OF DRAWINGS

[FIG. 1] FIG. 1 is an example of an optometry device provided in an optometry system.
[FIG. 2] FIG. 2 is a view showing a measurement unit.
[FIG. 3] FIG. 3 is a schematic configuration view of the inside of the optometry device when viewed from the front.
[FIG. 4] FIG. 4 is a schematic configuration view of the inside of the optometry device when viewed from the side.
[FIG. 5] FIG. 5 is a schematic configuration view of the inside of the optometry device when viewed from above.
[FIG. 6] FIG. 6 is a view showing a control system of the optometry device.
[FIG. 7] FIG. 7 is a view showing an example of a flow of a subjective examination on a subject eye.
[FIG. 8] FIG. 8 is an example of a monitor.
[FIG. 9] FIG. 9 is an example of a graph showing a relationship between a reaction time and a correction power in a refractive correction examination.
[FIG. 10] FIG. 10 is an example of a graph showing a relationship between a reaction time and a visual acuity value in a naked eye visual acuity examination.
[FIG. 11] FIG. 11 is an example of the optometry device.
[FIG. 12] FIG. 12 is a flowchart showing a detailed flow of a refractive correction examination.
[FIG. 13] FIG. 13 is an example of a flow of a visual acuity examination.

### DESCRIPTION OF EMBODIMENTS

### <Overview>

An overview of an optometry system according to an embodiment of the present disclosure will be described. The items classified by <> below can be used independently or in association with each other.

The optometry system in the present embodiment is an optometry system for subjectively measuring an optical characteristic of a subject eye. Examples of the optical characteristic of the subject eye may include at least one of the eye refractive power (for example, spherical power, cylindrical power, astigmatism axis angle, prism amount, and the like), a binocular vision function (for example, prism amount, stereoscopic vision function, and the like), contrast sensitivity, or the like of the subject eye.

The optometry system in the present embodiment may include at least a subjective optometry device (for example, an optometry device 100). For example, the subjective optometry device subjectively measures the optical characteristic of the subject eye by projecting the target light flux toward the subject eye and changing the optical characteristic of the target light flux. For example, the subjective optometry device may have a visual target presenting means that will be described later, a correction means that will be described later, and the like. Further, for example, the subjective optometry device may have a response input means that will be described later.

### <Visual target presenting means>

The optometry system in the present embodiment includes a visual target presenting means. The visual target presenting means emits a target light flux toward the subject eye.

For example, the visual target presenting means may be a display (for example, a display 31). Further, for example, a light source and a digital micromirror device (DMD) can be used as the visual target presenting means. Further, for example, the visual target presenting means may be a light source and a visual target plate.

For example, the target light flux from the visual target presenting means may be emitted directly toward the subject eye. Further, for example, the target light flux from the visual target presenting means may be indirectly guided toward the subject eye via a light projecting optical system (for example, a light projecting optical system 30). For example, the light projecting optical system may have at least one optical member for passing the target light flux emitted from the visual target presenting means. As an example, a lens, a mirror, and the like may be provided.

The optometry system may include a visual target presenting means as a member that partially configures the subjective optometry device. In this case, the subjective optometry device may be provided with the visual target presenting means. In addition, the optometry system may include a visual target presenting means as a visual target presentation device in addition to the subjective optometry device.

### <Correction means>

The optometry system in the present embodiment includes a correction means. For example, the correction means may include a correction optical system (for example, a correction optical system 60). For example, the correction optical system is arranged in the optical path of the light projecting optical system and changes the optical characteristic of the target light flux.

The correction optical system may have any configuration as long as it is possible to change the optical characteristic of the target light flux.

As an example, the correction optical system may include an optical element and control the optical element to change the optical characteristic of the target light flux. For example, the optical element may be at least one of a spherical lens, a cylindrical lens, a cross cylinder lens, a rotary prism, a wavefront modulation element, a varifocal lens, or the like. It is needless to say that the optical elements may differ from these.

Further, as an example, the correction optical system may optically change the presentation position (presentation distance) of the visual target with respect to the subject eye to change the optical characteristic of the target light flux. In this case, the visual target presenting means may be moved in the optical axis direction, or an optical element (for example, a spherical lens or the like) arranged in the optical path may be moved in the optical axis direction.

Further, for example, the correction optical system may arrange an optical element between the visual target presenting means and the optical member for guiding the target light flux from the light projecting optical system toward the subject eye and control the optical element to, change the optical characteristic of the target light flux. In other words, the correction optical system may be a phantom lens refractometer (phantom correction optical system).

Further, as an example, the correction optical system may switch and arrange optical elements arranged in front of the subject eye to change the optical characteristic of the target light flux. That is, the correction optical system may be an optometry unit (phoropter). Further, for example, the optometry unit may include a lens disk in which a plurality of optical elements are arranged on the same circumference, and a driving means for rotating the lens disk, and may electrically switch the optical elements by the drive of the driving means (for example, a motor).

### <Response input means>

The optometry system in the present embodiment includes the response input means. The response input means is a means for the examinee to input the answer obtained by reading the examination visual target. For example, the response input means may be an operation means such as a lever switch, or a push button switch (for example, an examinee controller 8). Further, the response input means may be a voice input means such as a microphone. In addition, the response input means may be a detection means that detects movement of the line of sight or gestures of the examinee.

### <Reaction time acquisition means>

The optometry system according to the present embodiment includes a reaction time acquisition means (for example, a controller 70). The reaction time acquisition means acquires a reaction time required from the start of the measurement to the response, based on the start timing at which the measurement of the subject eye is started, which is the start timing at which the visual target presenting means or the correction means is controlled by the control means (which will be described later), and the response timing after the start timing, which is the response timing at which the response signal for the measurement is input by the response input means (for example, a controller 6 and the examinee controller 8).

For example, the start timing at which the measurement of the subject eye is started may be the timing at which a start signal serving as a trigger for controlling the visual target presenting means or the correction means is acquired. For example, the start timing may be the timing at which the start signal is automatically input based on an optometry program or the like. Alternatively, for example, the start timing may be the timing at which the start signal is input by the examiner's operation of the operation means (for example, the controller 6). Alternatively, for example, the start timing may be the timing at which the start signal input by the examiner's operation of the operation means is received.

For example, such a start timing may be the timing at which measurement is started individually in the process of measuring the examination item for the subject eye. More specifically, the start timing may be the timing at which either the visual target presenting means or the correction means is sequentially controlled in the process of measuring the examination item. For example, the start timing may be each timing at which a start signal for switching the visual target is input or received or each timing at which the visual target is switched based on the start signal in the process of measuring the examination item. The timing at which the visual target is switched may be the timing at which the visual acuity value of the visual target is switched or the timing at which the type of the visual target is switched. In addition, for example, the start timing may be each timing at which a start signal for switching the correction power for correcting the subject eye is input or received or each timing at which the correction power is switched based on the start signal in the process of measuring the examination item.

Further, for example, such a start timing may be the timing at which the measurement of the examination items for the subject eye is started. More specifically, the start timing may be the timing at which either the visual target presenting means or the correction means is first controlled in order to start measuring the examination item. For example, the start timing may be the timing at which a start signal for switching the visual target is first input or received, in which the start timing may be the timing at which the visual target is first switched based on the start signal in the measurement of the examination items. Further, for example, the start timing may be the timing at which a start signal for switching the correction power is first input or received, in which the start timing may be the timing at which the correction power is first switched based on the start signal in the measurement of the examination items.

For example, the response timing at which the response signal for the measurement is input may be the timing at which the response signal is acquired based on the operation of the response input means. For example, the response timing may be the timing at which the response signal is input by operating the response input means. Further, for example, the response timing may be the timing at which the response signal input by operating the response input means is received. The response input means may be operated by the examiner or may be operated by the examinee. In a case where the examiner operates the response input means, the response input means may also serve as the operation means.

For example, such a response timing may be the timing at which each measurement ends individually in the process of measuring the examination item for the subject eye. More specifically, the response timing may be each timing at which either the visual target presenting means or the correction means is sequentially controlled and the response signal with respect to this is input in the process of measuring the examination item. For example, the response timing may be each timing at which a response signal is input in accordance with the switching of the visual target, in which the response timing may be each timing at which the response of the examinee is input in the process of measuring the examination item. Further, for example, the response timing may be each timing at which a response signal is input in accordance with the switching of the correction power, in which the response timing may be each timing at which the response of the examinee is input in the process of measuring the examination item.

Further, for example, the response timing at which the response signal for the measurement of the subject eye is input may be the timing at which the measurement of the examination items for the subject eye is ended. More specifically, the response timing may be the timing at which either the visual target presenting means or the correction means is last controlled and the response signal with respect to this is input, in which the response timing may be the timing at which the response of the examinee is input in the measurement of the examination items.

The examination items described above may be the entire examination of the subjective examination. Further, the examination items described above may be each examination item in the subjective examination. For example, at least one of the naked eye visual acuity examination, the refractive correction examination, or the like may be used. For example, the refractive correction examination may include a spherical examination, an astigmatism examination, or the like. For example, a spherical examination may include a red-green examination, or the like. For example, an astigmatism examination may include a cross-cylinder examination, or the like.

For example, the reaction time acquisition means may acquire the reaction time by measuring the time from the start timing to the response timing. That is, the reaction time acquisition means may acquire the reaction time by starting time measurement based on the above-described start signal and ending time measurement based on the above-described response signal.

As an example, the reaction time acquisition means may acquire each reaction time when sequentially switching at least one of the visual target or the correction power by starting time measurement at the start timing at which either the visual target presenting means or the correction means is controlled and ending time measurement at the response timing at which the response signal for this is input, in the process of measuring the examination item. That is, the reaction time may be acquired in real time during the examination.

Further, as an example, the reaction time acquisition means may acquire the entire response time required for the measurement of the examination items by starting time measurement at the start timing at which either the visual target presenting means or the correction means is first controlled and ending time measurement at the response timing at which the response signal is input in a case where either the visual target presenting means or the correction means is last controlled in the measurement of the examination items.

For example, the reaction time acquisition means may acquire the reaction time by calculating the difference between the start timing and the response timing. For example, the reaction time acquisition means may acquire the reaction time by starting time measurement at a predetermined timing, storing the time when the above-described start signal and the above-described response signal are obtained, and obtaining the difference between the start timing and the response timing.

As an example, the reaction time acquisition means may acquire each response time when sequentially switching at least one of the visual target and the correction power from the difference between the start timing at which either the visual target presenting means or the correction means is controlled and the response timing at which the response signal for this is input, in the process of measuring the examination item.

Further, as an example, the reaction time acquisition means may acquire the entire reaction time required for the measurement of the examination items from the difference between the start timing at which either the visual target presenting means or the correction means is first controlled and the response timing at which the response signal is input in a case where either the visual target presenting means or the correction means is last controlled, in the measurement of the examination items.

For example, the reaction time acquisition means may acquire the entire reaction time by adding the reaction times acquired for each examination item, in the process of measuring the examination items.

For example, the reaction time acquisition means may acquire a first reaction time and a second reaction time acquired at different timings, as the reaction time required from the start of measurement to the response. At least one of the start timing or the response timing may be different between the first reaction time and the second reaction time.

For example, the reaction time acquisition means may acquire the first reaction time and the second reaction time at different start timings. An example may be two or more reaction times in the process of measuring the examination item. In addition, an example may be the reaction time including the entire reaction time required for the measurement of the examination items and the reaction time excluding the start timing at which either the visual target presenting means or the correction means is first controlled.

Further, for example, the reaction time acquisition means may acquire the first reaction time and the second reaction time at different response timings. An example may be two or more reaction times in the process of measuring the examination item. In addition, an example may be the reaction time including the entire reaction time required for the measurement of the examination items and the reaction time excluding the response timing at which either the visual target presenting means or the correction means is last controlled.

Further, for example, the reaction time acquisition means may acquire the first reaction time and the second reaction time at different start timings and different response timings. An example may be two or more reaction times in the process of measuring the examination item. In addition, an example may be the reaction time including the entire reaction time required for the measurement of the examination items and the reaction time excluding the start timing at which either the visual target presenting means or the correction means is first controlled and the response timing at which either the visual target presenting means or the correction means is last controlled.

It is needless to say that, for example, the reaction time acquisition means may acquire a time different from the reaction time required from the start of measurement to the response. As an example, the reaction time acquisition means may acquire the examination time required for a series of measurements in the examination item. In this case, the reaction time acquisition means may acquire the examination time by adding the operation time for the operator to operate the response input means to the entire reaction time required for the measurement of the examination item. More specifically, for example, the examination time may be acquired by adding the time from each response timing to the next start timing in the process of measuring the examination item to the entire reaction time required for the measurement of the examination item.

### <Setting means>

The optometry system according to the present embodiment includes a setting means (for example, the controller 70). The setting means sets a reference value based on the reaction time of the examinee with respect to the examination visual target. In other words, a reference value as a reference for the time required for the examinee to respond using the response input means after visually recognizing the examination visual target (that is, reaction time), is set. For example, by the setting means, it is possible to change the reference value to a different reference value for each examinee in consideration of individual differences in reaction speed of the examinee.

For example, the setting means may set the reference value of the reaction time of the examinee based on a signal input by the operation of the examiner by the operation means. As an example, in this case, a list of a plurality of reference values may be displayed from which any time can be selected. Further, for example, the setting means may set the reference value of the reaction time of the examinee based on the examinee information. As an example, in this case, the reference value may be set using a table or the like that associates the examinee information with the reference value. For example, the table may be preset from the result of experiments, simulations, and the like. In addition, for example, the examinee information may be at least one of age, sex, and the like.

For example, the setting means may set the reaction time acquired by the reaction time acquisition means, as the reference value for the reaction time of the examinee. Further, for example, the setting means may set a time obtained by increasing or decreasing a predetermined time from the reaction time acquired by the reaction time acquisition means, as the reference value for the reaction time of the examinee. For example, the predetermined time may be preset from the result of experiments, simulations, and the like.

For example, the setting means may be capable of setting a reference value based on the reaction time of the examinee according to the examination item. That is, the reference value for the reaction time may be set according to the refractive correction examination (red-green examination, cross-cylinder examination, and the like) or the visual acuity examination. For example, in this case, the setting means may set the reference value in consideration of different examination visual targets for each examination item. As an example, the larger the number of examination visual targets to be presented simultaneously, the larger the reference value (longer the time) may be set. It is needless to say that the reference value may be set in consideration of the complexity of the examination, the number of answer patterns, and the like.

For example, the setting means may change the reference value for the reaction time of the examinee from the first reference value to the second reference value, and set the reference value, based on the reaction time acquired by the reaction time acquisition means in real time. For example, the second reference value may be a value different from the first reference value. For example, the second reference value may be a value greater than the first reference value (longer time) or a value smaller than the first reference value (shorter time). That is, the setting means may update the reference value which is a reference of the reaction time of the examinee, based on the real-time reaction time obtained in a case where at least one of the examination visual target or the correction power is sequentially switched during the examination of the subject eye. In other words, the reference value may be optimized as appropriate during the examination of the subject eye. Accordingly, the reference value of the examinee is changed each time, according to the change of the reaction speed of the examinee.

In such a case, the setting means may optimize and set the reference value, based on the number of times of acquisition of the reaction time acquired by the reaction time acquisition means. For example, the reference value may be updated each time the reaction time acquisition means acquires the reaction time (that is, each time). Further, for example, a plurality of reaction times acquired by the reaction time acquisition means may be averaged and reflected in the reference value.

### <State acquisition means>

The optometry system according to the present embodiment includes a state acquisition means (for example, the controller 70). The state acquisition means acquires the state of the optometry system in a case where the visual target presenting means or the correction means is controlled by the control means which will be described later.

For example, the state acquisition means may acquire the state of the visual target presenting means in a case where the visual target presenting means is controlled by the control means. Further, for example, the state acquisition means may acquire the state of the correction means in a case where the visual target presenting means is controlled by the control means. Further, for example, the state acquisition means may acquire the state of the visual target presenting means in a case where the correction means is controlled by the control means. Further, for example, the state acquisition means may acquire the state of the correction means in a case where the correction means is controlled by the control means which will be described later. It is needless to say that the state which combined these may be acquired.

For example, the state of the visual target presenting means may include at least one of the state of the type of the visual target, the state of the visual acuity value of the visual target, or the like, which is presented to the subject eye by the control means controlling the visual target presenting means. For example, the state of the visual target presenting means may include the number of times (frequency) of switching the type of the visual target, the number of times (frequency) of switching the visual acuity value of the visual target, and the like. Further, for example, the state of the correction means may be the state of the correction power of the subject eye, which is obtained by correcting the subject eye by the control means controlling the correction means. For example, the state of the correction means may include the number of times (frequency) of switching correction power.

### <Determination means>

The optometry system according to the present embodiment includes a determination means (for example, the controller 70). The determination means determines the suitability of the state of the optometry system acquired by the state acquisition means, based on the reaction time acquired by the reaction time acquisition means.

For example, the determination means may determine the suitability of the state of the optometry system, based on whether or not the reaction time exceeds a preset first threshold value. For example, the first threshold value of the reaction time may be set from experiments, simulations, and the like. Further, for example, the first threshold value of the reaction time may be set based on the average human reaction speed. In this case, the first threshold value of the reaction time may be set for each age. It is needless to say that the first threshold value of the reaction time may be set for each item different from age. Further, for example, the first threshold value of the reaction time may be set based on the reaction speed of each examinee (that is, the reference value for the reaction time may be set for each examinee). An allowable range may be provided for the first threshold value of the reaction time.

For example, the determination means may determine whether or not the subject eye is being properly examined, as the suitability of the state of the optometry system, based on the reaction time acquired by the reaction time acquisition means. As an example, the determination means may determine that the subject eye is being properly examined by estimating that the examinee see the visual target correctly based on the reaction time. More specifically, for example, in a case where the reaction time is approximately the same as the first threshold value, the state of the visual target presenting means and the state of the correction means are appropriate, and thus it may be determined that the examinee can easily recognize the visual target.

Further, as an example, the determination means may determine that the subject eye is not being properly examined by estimating that the examinee is confused about the answer based on the reaction time. More specifically, for example, in a case where the reaction time is longer than the first threshold value, it is estimated that the examinee is confused since the examinee answer slowly, at least one of the state of the visual target presenting means or the state of the correction means is inappropriate, and thus it may be determined that the visual target cannot be recognized.

Further, as an example, the determination means may determine that the subject eye has not been properly examined by estimating that the examinee gives a correct answer by chance based on the reaction time. More specifically, for example, in a case where the reaction time is shorter than the first threshold value, it is estimated that the examinee has answered by feeling since the examinee answered quickly, at least one of the state of the visual target presenting means or the state of the correction means is inappropriate, and thus it may be determined that the visual target cannot be recognized.

For example, the determination means may determine the success or failure of the response of the examinee based on whether or not the reaction time exceeds a preset second threshold value. For example, the first threshold value for determining the suitability of the state of the optometry system and the second threshold value for determining the success or failure of the response of the examinee may be the same or may be different. For example, in a case where the first threshold value and the second threshold value are different, the second threshold value may be set in a direction of making the reaction time shorter with respect to the first threshold value, and may be set in a direction of making the reaction time longer with respect to the first threshold value. It is needless to say that the second threshold value may be set both in the direction of making the reaction time shorter and the direction of making the reaction time longer with respect to the first threshold value.

For example, the determination means may change the determination method of the success or failure of the response of the examinee, based on whether or not the reaction time exceeds a preset second threshold value. As an example, the determination means may determine that the response of the examinee is successful in a case where the reaction time does not exceed the second threshold value. At this time, further, the determination means may determine that the response of the examinee is successful in a case where the response of the examinee is correct, and may determine that the response of the examinee fails in a case where the response of the examinee is incorrect. Further, as an example, the determination means may determine that the response of the examinee is a failure in a case where the reaction time exceeds the second threshold value. At this time, the determination means may further determine that the response of the examinee is a failure regardless of whether the response of the examinee is correct or incorrect.

### <Control means>

The optometry system according to the present embodiment includes control means (for example, the controller 70). The control means controls the operation of the optometry system. For example, the control means may control the operation of the optometry system, based on the start signal for controlling the operation of the optometry system. In this case, the control means may control the operation of the optometry system, based on the start signal automatically input based on the optometry program or the like. Further, in this case, the control means may control the operation of the optometry system, based on a start signal input by the examiner's operation of the operation means (for example, the controller 6). Further, in this case, the control means may control the operation of the optometry system, based on the start signal received by the examiner's operation of the operation means. It is needless to say that, for example, the control means may control the operation of the optometry system, based on a start signal (input signal) input by the operation of the response input means by the examinee. Further, for example, the control means may control the operation of the optometry system, based on a start signal (input signal) received by the operation of the response input means by the examinee. For example, an examination (optometry) of a subject eye may be automatically proceeded based on these start signals.

For example, the optometry program may include a self-optometry application program. The self-optometry application program is a program for realizing an application (self-optometry application) that automatically proceeds with the optometry based on the answers input by the examinee. In this case, the control means may control the operation of the optometry system by executing a self-optometry application.

For example, the control means can control at least one of the visual target presenting means or the correction means. For example, the control means can control the display of the visual target presenting means to change at least one of the type of the visual target, the visual acuity value of the visual target, or the like. Further, as an example, the control means can control the correction optical system and change at least one of the correction power that corrects the subject eye, the arrangement of the optical elements, or the like. It is needless to say that, for example, the control means may be enabled to control a means other than the visual target presenting means and the correction means.

For example, the control means may control the operation of the optometry system, based on the reaction time acquired by the reaction time acquisition means. As an example, the control means may change the visual acuity value of the visual target in a case where a predetermined reaction time has elapsed. Further, as an example, the correction power may be changed in a case where a predetermined reaction time has elapsed. That is, the control means may automatically switch the optometry system to the next setting after a predetermined reaction time has elapsed. Accordingly, the examination content is optimized and the examination time is shortened.

For example, the control means may control the operation of the optometry system, based on the determination result of the determining the suitability of the state of the optometry system by the determination means. For example, the control means may control the operation of the optometry system each time a determination result indicating the suitability of the state of the optometry system is obtained. Further, for example, the control means may control the operation of the optometry system in a case where the determination result indicating the suitability of the state of the optometry system becomes the same for a predetermined number of times in succession.

For example, the control means may change the state of the visual target presenting means (that is, the state of the visual acuity value of the visual target and the like), based on the suitability of the state of the optometry system. As an example, the control means may change the number of steps for changing the visual acuity value of the visual target, based on the suitability of the state of the optometry system. More specifically, the control means may change the visual acuity value of the visual target by two steps in a case where the state of the optometry system is appropriate, and may change the visual acuity value of the visual target by one step in a case where the state of the optometry system is inappropriate.

Further, for example, the control means may change the state of the correction means (that is, the state of the correction power and the like), based on the suitability of the state of the optometry system. As an example, the control means may change the number of steps for changing the correction power, based on the suitability of the state of the optometry system. More specifically, the control means may change the correction power by one step (0.25 D) in a case where the state of the optometry system is appropriate, and may change the correction power by two steps (0.5 D) in a case where the state of the optometry system is inappropriate.

For example, the control means may control the operation of the optometry system, based on the determination result made by the determination means regarding the success or failure of the response of the examinee. For example, the control means may control the operation of the optometry system as described above in a case where it is determined that the response of the examinee is valid. Further, for example, the control means may not necessarily control the operation of the optometry system in a case where it is determined that the response of the examinee is invalid. That is, the control means may control the operation of the optometry system by using the reaction time at which the response of the examinee is valid and excluding the reaction time at which the response of the examinee is determined to be invalid.

### <Output means>

The optometry system according to the present embodiment includes an output means (for example, the controller 70). The output means outputs the reaction time acquired by the reaction time acquisition means. For example, by outputting the reaction time required by the examinee from the start of the examination to the response, it is possible to estimate the possibility that the examinee has given a correct answer by chance, and to determine whether or not the examination result of the subject eye is accurate. In addition, it is possible to easily determine the setting of the next examination, and the like, in consideration of the possibility that the examinee has given a correct answer by chance.

For example, the output means may cause the display means to display the reaction time by outputting the reaction time to the display means (for example, a monitor 6a). Further, for example, the output means may generate the reaction time as voice guide by outputting the reaction time to the voice generation means (for example, a speaker). In addition, for example, the output means may print the reaction time by outputting the reaction time to a printing means (for example, a printer). Further, for example, the output means may store the reaction time by outputting the reaction time to a storage means (for example, a memory, a server, and the like).

For example, the output means may output the reaction time in at least one of these output forms. It is needless to say that, for example, the output means may output the identifier associated with the reaction time in at least one of these output forms. For example, a character string, a one-dimensional code, a two-dimensional code, or the like may be used for output.

For example, the output means may output the first reaction time and the second reaction time acquired by the reaction time acquisition means in a comparable manner. As an example, the output means may output the first reaction time and the second reaction time side by side, or output these in a switchable manner. In addition, as an example, the output means may output the first reaction time and the second reaction over time. Accordingly, it becomes easier to understand the probability that the examinee has given a correct answer by chance, from the change in the reaction time of the examinee.

For example, the output means may output information including at least reaction time. As an example, the output means may output the reaction time itself. In other words, the number of seconds required for the examinee to respond from the start of measurement may be output. Further, as an example, the output means may output the state of the optometry system acquired by the state acquisition means, together with the reaction time. More specifically, along with the reaction time, the state of the visual target presenting means (at least one state of the type of the visual target, the visual acuity value of the visual target, the number of times of switching the type of the visual target, the number of times of switching the visual acuity value of the visual target, or the like), the state of the correction means (at least one of the correction power of the subject eye, the number of times of switching the correction power of the subject eye, or the like), and the like may be output. Accordingly, it is easy to determine whether or not the reaction time of the examinee has increased or decreased according to changes in the state of the optometry system.

For example, the output means may output data that makes it possible to understand the relationship between the reaction time and the state of the optometry system. For example, data may be output that continuously indicates changes in reaction time with changes in the state of the optometry system. As an example, at least one of graph data, table data, or the like, which continuously show such changes, may be output.

For example, the output means may output the reaction time and the state of the optometry system, as graph data showing the relationship between the reaction time and the state of the optometry system. As an example, the output means may output graph data showing the relationship between the reaction time and the visual acuity value of the visual target set by the visual target presenting means. Further, as an example, the output means may output graph data showing the relationship between the reaction time and the correction power set by the correction means. Accordingly, the change in reaction time of the examinee according to the state of the optometry system can be easily understood.

It is needless to say that, for example, the output means may output information different from the state of the optometry system together with the reaction time. For example, at least one of information such as the subjective value of the subject eye measured by the subjective examination, the examination time required for a series of measurements in each examination item, and the like may be output.

### <Leading information output means>

The optometry system according to the present embodiment includes a leading information output means (for example, the controller 70). The leading information output means outputs leading information for leading the examinee to input an answer, based on the reference value set by the setting means, during the examination of the subject eye. For example, the leading information output means may output the leading information during the examination, based on the deviation amount of the measurement time with respect to the reference value. As a result, the hesitation of the examinee in the operation of self-optometry can be reduced, and accurate examination results can be obtained. In addition, for example, accordingly, the leading information is output at an appropriate timing for each examinee, the burden on the examinee is reduced, and the examination can proceed properly. In other words, in consideration of the reaction speed that differs for each examinee, it is possible to prompt the examinee to input an answer at a timing that is neither too early nor too late according to the examinee.

For example, the leading information output means may control the display means (for example, a display 31), to display the leading information on the display means. Further, for example, the leading information output means may control the voice generation means (for example, a speaker), to cause the voice generation means to generate the leading information as voice. The leading information output means may execute control combining these, or may execute control different from these.

For example, the leading information output means may repeatedly output the leading information in a case where no signal is obtained from the response input means even when a predetermined time set for repeating the leading information has elapsed. In this case, the predetermined time may be set to any time by the examiner. In addition, in this case, the predetermined time may be set to a time set in advance based on experiments, simulations, and the like. Further, in this case, the predetermined time may be a time based on a reference value. For example, the time may be the same as the reference value, or the time obtained by increasing or decreasing the reference value by a predetermined amount of time.

For example, at this time, the leading information may always be repeatedly output at predetermined time. That is, the leading information may be repeatedly output at constant time intervals. Further, for example, at this time, the leading information may be repeatedly output at any time. As an example, the output may be repeatedly output at time interval that becomes shorter or longer step by step. Further, as an example, the leading information may be repeatedly output such that the time interval changes according to the number of times of output of the leading information. As a result, the examination can be easily proceeded even when the examinee forget the operation method of the response input means during the examination.

When repeating the leading information, the leading information output means may change the leading information from the first leading information to the second leading information and output the leading information according to the number of times of output of the leading information. For example, the second leading information may be information at least partially different from the first leading information. As an example, the first leading information may be information representing the operation method for operating the response input means in order for the examinee to input an answer to the visually recognized examination visual target. Further, as an example, the second leading information may be information representing an operation method for operating the response input means in order for the examinee to call the examiner. Further, the second leading information may be information including information representing the operation method for the examinee to input an answer and information representing the operation method for calling the examiner. It is needless to say that the first leading information and the second leading information may be information different from these.

For example, the number of times of output of the leading information may be a number set in any manner by the examiner. Further, for example, the number of times of output of the leading information may be a number preset based on experiments, simulations, and the like. For example, the leading information may be switched every predetermined number of times of outputs. As an example, in a case where the number of times of output is set to two, the second leading information may be output twice after the first leading information is output twice. The first leading information and the second leading information may be alternately output based on the number of times of output of the leading information. It is needless to say that the information is not limited to the first leading information and the second leading information, and may further include third leading information, fourth leading information, and the like, and each output may be switched.

The present disclosure is not limited to the device described in the present embodiment. For example, terminal control software (program) that performs the functions of the embodiment described below is supplied to the system or device via a network or various storage media, and the control device (for example, CPU or the like) of the system or device can also read and execute the program.

### <First example>

A first example of the optometry system according to the present embodiment will be described.

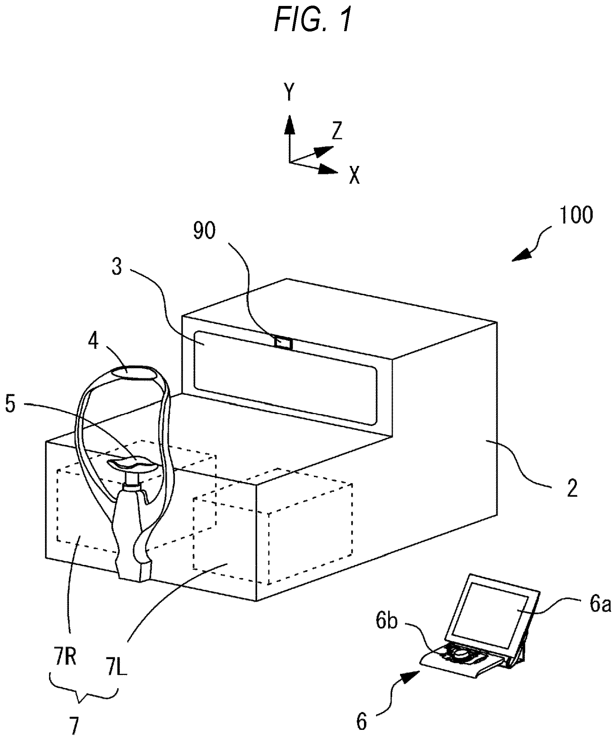

FIG. 1 is an example of a subjective optometry device 100 included in an optometry system 1. For example, the subjective optometry device 100 (hereinafter referred to as the optometry device 100) can subjectively measure optical characteristic of a subject eye. For example, as the optical characteristic of the subject eye, subjective eye refractive power (that is, subjective value) of the subject eye can be measured. For example, the optometry device 100 includes a housing 2, a presentation window 3, a forehead pad 4, a chin support 5, a controller 6, an imaging unit 90, and the like.

The presentation window 3 is used to present the visual target to a subject eye E. The forehead pad 4 is used to keep the distance between the subject eye E and the optometry device 100 constant. The chin support 5 is used to keep the distance between the subject eye E and the optometry device 100 constant.

The controller 6 includes a monitor 6a, a switch unit 6b, and the like. The monitor 6a displays various types of information (for example, the measurement result of the subject eye E and the like). The monitor 6a is a touch panel, and the monitor 6a also functions as the switch unit 6b. The switch unit 6b is used to make various settings (for example, input of a start signal and the like). The signal corresponding to the operation instruction from the controller 6 is output to the controller 70 by wired communication through a cable which is not shown. It is needless to say that the signal corresponding to the operation instruction from the controller 6 may be output to the controller 70 by wireless communication through infrared rays or the like.

The imaging unit 90 includes an imaging optical system which is not shown. For example, the imaging optical system is used to image a face of an examinee. For example, the imaging optical system may include an imaging element and a lens.

### <Measurement unit>

The measurement unit 7 includes a left eye measurement unit 7L and a right eye measurement unit 7R. In the present example, the left eye measurement unit 7L and the right eye measurement unit 7R are configured with the same members. It is needless to say that at least a part of the left eye measurement unit 7L and the right eye measurement unit 7R are configured with different members. The measurement unit 7 has a pair of left and right subjective measurement units which will be described later, and a pair of left and right objective measurement units which will be described later. The target light flux and the measured light flux from the measurement unit 7 are guided to the subject eye E through the presentation window 3.

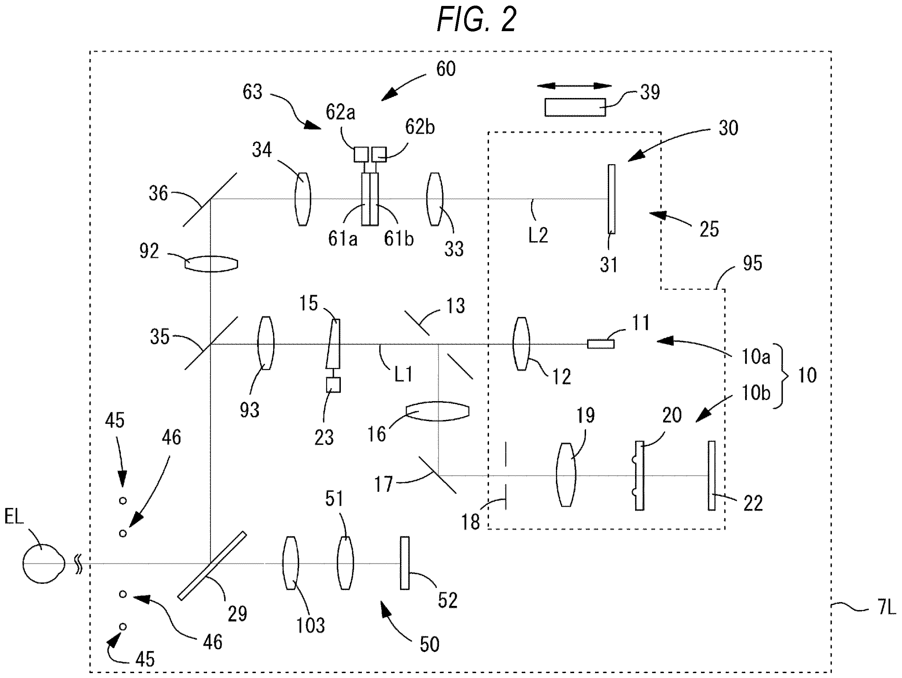

FIG. 2 is a view showing the measurement unit 7. In FIG. 2, as the measurement unit 7, the left eye measurement unit 7L is taken as an example. Since the right eye measurement unit 7R has the same configuration as that of the left eye measurement unit 7L, the description thereof will be omitted. For example, the left eye measurement unit 7L includes an objective measurement optical system 10, a subjective measurement optical system 25, a first index projection optical system 45, a second index projection optical system 46, an observation optical system 50, and the like.

### <Objective measurement optical system>

The objective measurement optical system 10 is used as a part of the configuration of the objective measurement unit that objectively measures the optical characteristic of the subject eye. In the present example, an objective measurement unit that measures the eye refractive power of the subject eye E, as the optical characteristic of the subject eye E, will be described as an example. In addition to the eye refractive power, the optical characteristic of the subject eye E may be the eye axial length, the corneal shape, and the like. For example, the objective measurement optical system 10 includes a projection optical system 10a and a light receiving optical system 10b.

The projection optical system 10a projects a spot-shaped measurement index onto the fundus of the subject eye E through the pupil center portion of the subject eye E. For example, the projection optical system 10a includes a light source 11, a relay lens 12, a hole mirror 13, a prism 15, an objective lens 93, a dichroic mirror 35, a dichroic mirror 29, and the like.

The light receiving optical system 10b takes out the fundus reflected light flux reflected by the fundus of the subject eye E in a ring shape, through the peripheral portion of the pupil of the subject eye E. For example, the light receiving optical system 10b includes the dichroic mirror 29, the dichroic mirror 35, the objective lens 93, the prism 15, the hole mirror 13, a relay lens 16, a mirror 17, a light receiving diaphragm 18, a collimator lens 19, a ring lens 20, an imaging element 22, and the like.

In the present example, the description of the projection optical system 10a and the light receiving optical system 10b will be omitted. For details of these, refer to, for example, JP2018-047049A.

### <Subjective measurement optical system>

The subjective measurement optical system 25 is used as a part of the configuration of the subjective measurement unit that subjectively measures the optical characteristic of the subject eye E. In the present example, a subjective measurement unit for measuring the eye refractive power of the subject eye E, as the optical characteristic of the subject eye E, is taken as an example. In addition to the eye refractive power, the optical characteristic of the subject eye E may be the contrast sensitivity, the binocular vision function (for example, the amount of phoria, the stereoscopic vision function, and the like), and the like. For example, the subjective measurement optical system 25 includes the light projecting optical system 30 and the correction optical system 60.

### <Light projecting optical system>

The light projecting optical system 30 projects a target light flux toward the subject eye E. For example, the light projecting optical system 30 includes the display 31, a projective lens 33, a projective lens 34, a reflection mirror 36, an objective lens 92, the dichroic mirror 35, the dichroic mirror 29, and the like.

A visual target (fixation target, examination visual target, and the like) is displayed on the display 31. The target light flux emitted from the display 31 is projected onto the subject eye E through the optical members from the projective lens 33 to the dichroic mirror 29 in order. The dichroic mirror 35 makes the optical path of the objective measurement optical system 10 and the optical path of the subjective measurement optical system 25 a common optical path. In other words, the dichroic mirror 35 makes an optical axis L1 of the objective measurement optical system 10 and an optical axis L2 of the subjective measurement optical system 25 coaxial with each other. The dichroic mirror 29 is an optical path branching member. The dichroic mirror 29 reflects the target light flux projected by the light projecting optical system 30 and the measured light flux projected by the projection optical system 10a which will be described later, and guides the target light flux and the measured light flux to the subject eye E.

### <Correction optical system>

The correction optical system 60 is arranged in the optical path of the light projecting optical system 30. Further, the correction optical system 60 changes the optical characteristic of the target light flux emitted from the display 31. For example, the correction optical system 60 includes an astigmatism correction optical system 63, a drive mechanism 39 which will be described later, and the like.

The astigmatism correction optical system 63 is used to correct the cylindrical power and the astigmatism axis angle of the subject eye E. The astigmatism correction optical system 63 is arranged between the projective lens 33 and the projective lens 34. The astigmatism correction optical system 63 includes two positive lenses, a cylindrical lens 61a and a cylindrical lens 61b, having the same focal length. Each of the cylindrical lens 61a and the cylindrical lens 61b rotates independently around the optical axis L2 by driving a rotation mechanism 62a and a rotation mechanism 62b.

In the present example, a configuration in which the cylindrical lens 61a and the cylindrical lens 61b are used as the astigmatism correction optical system 63 has been described as an example, but the present invention is not limited thereto. The astigmatism correction optical system 63 may have a configuration capable of correcting the cylindrical power, the astigmatism axis angle, and the like. As an example, the corrective lens may be taken in and out of the optical path of the light projecting optical system 30.

### <Driving unit>

In the present example, the light source 11 and the relay lens 12 included in the projection optical system 10a, the light receiving diaphragm 18, the collimator lens 19, a ring lens 20, and the imaging element 22 included in the light receiving optical system 10b, , and the display 31 included in the light projecting optical system 30 can be integrally moved in the optical axis direction by the drive mechanism 39. In other words, the display 31, the light source 11, the relay lens 12, the light receiving diaphragm 18, the collimator lens 19, the ring lens 20, and the imaging element 22 are synchronized as a driving unit 95, and these are integrally moved by the drive mechanism 39. The drive mechanism 39 includes a motor and a slide mechanism. The moving position where the drive mechanism 39 has moved is detected by a potentiometer which is not shown.

The drive mechanism 39 moves the display 31 in the optical axis L2 direction by moving the driving unit 95 in the optical axis direction. Accordingly, in the objective measurement, cloud fog can be applied to the subject eye E. In the subjective measurement, the presentation distance of the visual target with respect to the subject eye E can be optically changed to correct the spherical power of the subject eye E. In other words, the configuration in which the display 31 is moved in the optical axis L2 direction is used as a spherical correction optical system that corrects the spherical power of the subject eye E, and the spherical power of the subject eye E is corrected by changing the position of the display 31. The configuration of the spherical correction optical system may be different from that of the present example. For example, the spherical power may be corrected by arranging multiple optical elements in the optical path. Further, for example, the spherical power may be corrected by arranging the lens in the optical path and moving the lens in the optical axis direction.

Further, the drive mechanism 39 moves the light source 11, the relay lens 12, and the members from the light receiving diaphragm 18 to the imaging element 22 in the optical axis L1 direction by moving the driving unit 95 in the optical axis direction. Accordingly, the light source 11, the light receiving diaphragm 18, and the imaging element 22 are arranged to be optically conjugated with respect to the fundus of the subject eye E. Regardless of the movement of the driving unit 95, the hole mirror 13 and the ring lens 20 are arranged to be conjugated with the pupil of the subject eye E at a constant magnification. Therefore, the fundus reflected light flux that is the reflection of the measured light flux of the projection optical system 10a is always incident on the ring lens 20 of the light receiving optical system 10b as a parallel light flux, and a ring-shaped light flux having the same size as the ring lens 20 is imaged on the imaging element 22 in a focused state, regardless of the eye refractive power of the subject eye E.

### <First index projection optical system and second index projection optical system>

The first index projection optical system 45 and the second index projection optical system 46 are arranged between the dichroic mirror 29 and a deflection mirror 81 which will be described later. The first index projection optical system 45 emits near infrared light for projecting an infinite-distance alignment index onto the cornea of the subject eye E. The second index projection optical system 46 is arranged at a position different from that of the first index projection optical system 45, and emits near infrared light for projecting a finitedistance alignment index onto the cornea of the subject eye. The near infrared light (alignment light) emitted from the second index projection optical system 46 is also used as the anterior eye part photographing light for photographing the anterior eye part of the subject eye by the observation optical system 50.

### <Observation optical system>

The observation optical system (imaging optical system) 50 includes the dichroic mirror 29, an objective lens 103, an imaging lens 51, an imaging element 52, and the like. The dichroic mirror 29 transmits the anterior eye part observation light and the alignment light. The imaging element 52 has an imaging surface arranged at a position conjugated with the anterior eye part of the subject eye E. The output from the imaging element 52 is input to the controller 70. Accordingly, the anterior eye part image of the subject eye E is imaged by the imaging element 52 and displayed on the monitor 6a. The observation optical system 50 also serves as an optical system that detects the alignment index image formed on the cornea of the subject eye E by the first index projection optical system 45 and the second index projection optical system 46, and the position of the alignment index image is detected by the controller 70.

### <Internal configuration of optometry device>

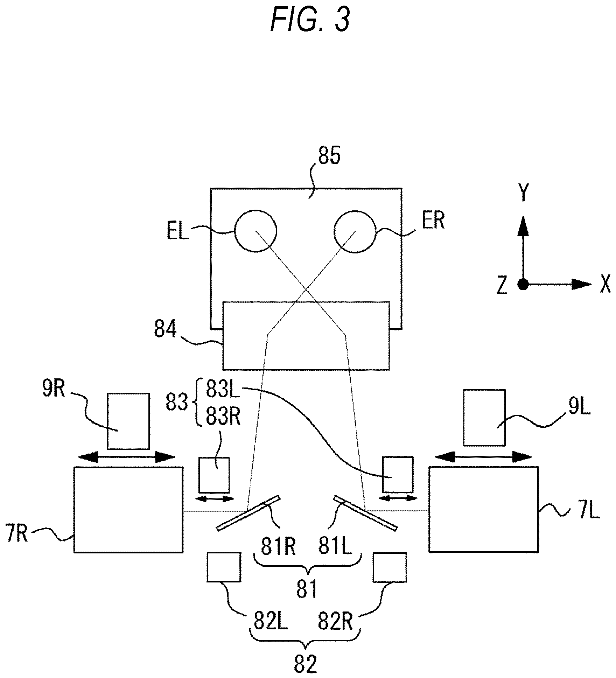

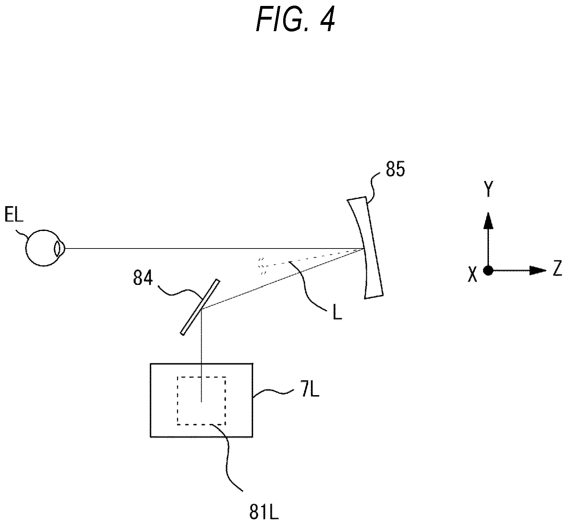

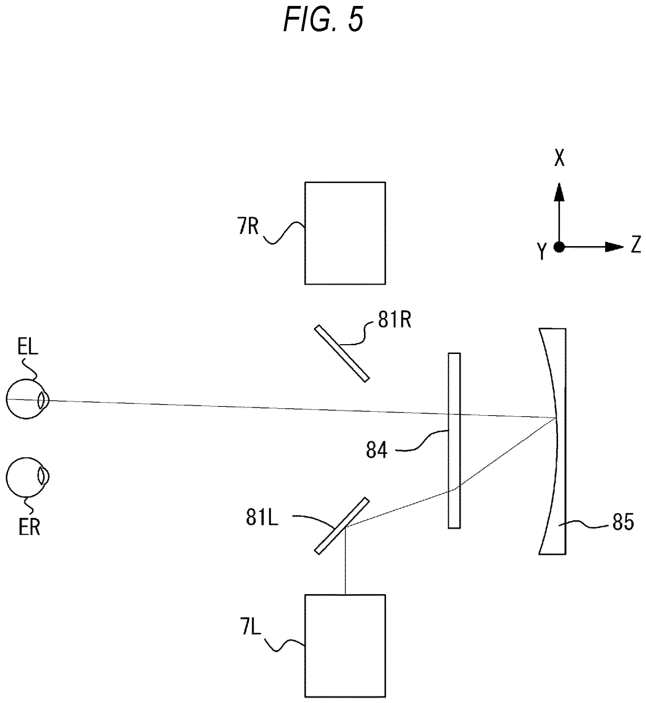

Next, the internal configuration of the optometry device 100 will be described. FIG. 3 is a schematic configuration view of the inside of the optometry device 100 when viewed from the front. FIG. 4 is a schematic configuration view of the inside of the optometry device 100 when viewed from the side. FIG. 5 is a schematic configuration view of the inside of the optometry device 100 when viewed from above. FIGS. 4 and 5 show only the optical axis of the left eye measurement unit 7L for convenience of description.

The optometry device 100 includes the subjective measurement unit. For example, the subjective measurement unit includes the measurement unit 7, the deflection mirror 81, a drive mechanism 82, a driving unit 83, a reflection mirror 84, a concave mirror 85, and the like. A configuration of the subjective measurement unit is not limited thereto. For example, the configuration may not include the reflection mirror 84. In this case, the target light flux from the measurement unit 7 may be emitted in the oblique direction with respect to the optical axis L of the concave mirror 85 after passing through the deflection mirror 81. Further, for example, a configuration of the subjective measurement unit may include a half mirror. In this case, the target light flux from the measurement unit 7 may be emitted in the oblique direction with respect to the optical axis L of the concave mirror 85 through the half mirror, and the reflected light flux may be guided to the subject eye E.

The optometry device 100 includes a left eye driving unit 9L and a right eye driving unit 9R. The left eye measurement unit 7L and the right eye measurement unit 7R can be moved in the X direction. For example, by moving the left eye measurement unit 7L and the right eye measurement unit 7R, the distance between the measurement unit 7 and the deflection mirror 81 which will be described later changes, and the presentation position of the target light flux from the measurement unit 7 in the Z direction is changed. Accordingly, the target light flux corrected by the correction optical system 60 is guided to the subject eye E, and the measurement unit 7 is adjusted in the Z direction such that an image of the target light flux corrected by the correction optical system 60 is formed on the fundus of the subject eye E.

For example, the deflection mirror 81 includes a right eye deflection mirror 81R and a left eye deflection mirror 81L, which are each provided in left and right pair. For example, the deflection mirror 81 is arranged between the correction optical system 60 and the subject eye E. In other words, the correction optical system 60 according to the present example includes a left eye correction optical system and a right eye correction optical system, which are each provided as a left and right pair, the left eye deflection mirror 81L is arranged between the left eye correction optical system and a left eye EL, and the right eye deflection mirror 81R is arranged between the right eye correction optical system and a right eye ER. For example, the deflection mirror 81 is preferably arranged at the pupil conjugate position.

For example, the left eye deflection mirror 81L reflects the light flux projected from the left eye measurement unit 7L and guides the light flux to the left eye EL. Further, for example, the left eye deflection mirror 81L reflects the fundus reflected light flux from the left eye EL and guides the fundus reflected light flux to the left eye measurement unit 7L. For example, the right eye deflection mirror 81R reflects the light flux projected from the right eye measurement unit 7R and guides the light flux to the right eye ER. Further, for example, the right eye deflection mirror 81R reflects the fundus reflected light flux from the right eye ER and guides the fundus reflected light flux to the right eye measurement unit 7R. In the present example, a configuration in which the deflection mirror 81 is used as a deflection member that reflects the light flux projected from the measurement unit 7 and guides the light flux to the subject eye E, has been described as an example, but the present invention is limited thereto. The deflection member may be, for example, a prism, a lens, or the like, as long as the deflection member can reflect the light flux projected from the measurement unit 7 and guide the light flux to the subject eye E.

For example, the drive mechanism 82 includes a motor (driving unit) and the like. For example, the drive mechanism 82 includes a drive mechanism 82L for driving the left eye deflection mirror 81L and a drive mechanism 82R for driving the right eye deflection mirror 81R. For example, the deflection mirror 81 rotates and moves by driving the drive mechanism 82. For example, the drive mechanism 82 rotates the deflection mirror 81 with respect to the rotation axis in the horizontal direction (X direction) and the rotation axis in the vertical direction (Y direction). In other words, the drive mechanism 82 rotates the deflection mirror 81 in the XY directions. The rotation of the deflection mirror 81 may be in either the horizontal direction or the vertical direction.

For example, the driving unit 83 includes a motor or the like. For example, the driving unit 83 includes a driving unit 83L for driving the left eye deflection mirror 81L and a driving unit 83R for driving the right eye deflection mirror 81R. For example, the deflection mirror 81 moves in the X direction by driving the driving unit 83. For example, by moving the left eye deflection mirror 81L and the right eye deflection mirror 81R, the distance between the left eye deflection mirror 81L and the right eye deflection mirror 81R is changed, and the distance in the X direction between the left eye optical path and the right eye optical path can be changed according to the pupillary distance of the subject eyes E.

For example, a plurality of deflection mirrors 81 may be provided in each of the left eye optical path and the right eye optical path. For example, a configuration in which two deflection mirrors are provided in each of the left eye optical path and the right eye optical path (for example, a configuration in which two deflection mirrors are provided in the left eye optical path), can be employed. In this case, one deflection mirror may be rotated in the X direction and the other deflection mirror may be rotated in the Y direction. For example, by rotating and moving the deflection mirror 81, the apparent light flux for forming an image of the target light flux in front of the subject eye is deflected, and the formation position of the image of the target light flux can be optically corrected.

For example, the concave mirror 85 is shared by the left eye measurement unit 7L and the right eye measurement unit 7R. For example, the concave mirror 85 is shared by the left eye optical path including the left eye correction optical system and the right eye optical path including the right eye correction optical system. In other words, the concave mirror 85 is arranged at a position that passes through both the left eye optical path including the left eye correction optical system and the right eye optical path including the right eye correction optical system. It is needless to say that the concave mirror 85 may not be shared by the left eye optical path and the right eye optical path. In other words, a concave mirror may be provided in each of the left eye optical path including the left eye correction optical system and the right eye optical path including the right eye correction optical system. For example, the concave mirror 85 guides the target light flux that has passed through the correction optical system 60 to the subject eye E, and forms an image of the target light flux that has passed through the correction optical system 60 in front of the subject eye E.

### <Optical path of subjective measurement unit>

The optical path of the subjective measurement unit will be described by taking the left eye optical path as an example. The right eye optical path has the same configuration as that of the left eye optical path. For example, in the subjective measurement unit for the left eye, in a case where the target light flux emitted from the display 31 in the subjective measurement optical system 25 is incident on the astigmatism correction optical system 63 through the projective lens 33, and passes through the astigmatism correction optical system 63, the target light flux passes through the projective lens 34, the reflection mirror 36, the objective lens 92, the dichroic mirror 35, and the dichroic mirror 29, and is guided toward the left eye deflection mirror 81L from the left eye measurement unit 7L. The target light flux reflected by the left eye deflection mirror 81L is reflected by the reflection mirror 84 toward the concave mirror 85. The target light flux emitted from the display 31 reaches the left eye EL through each optical member in this manner.

Accordingly, an image of the target light flux corrected by the correction optical system 60 is formed on the fundus of the left eye EL with reference to the spectacles wearing position (for example, approximately 12 mm from the corneal apex position) of the left eye EL. Therefore, the adjustment of the spherical power by the spherical power correction optical system (in the present example, the drive of the drive mechanism 39) in front of the eyes, is equivalent to the fact that the astigmatism correction optical system 63 is arranged as if the astigmatism correction optical system 63 is in front of the eye. In a natural state, the examinee can collimate the image of the target light flux formed in front of the eye optically at a predetermined examination distance through the concave mirror 85.

### <Controller>

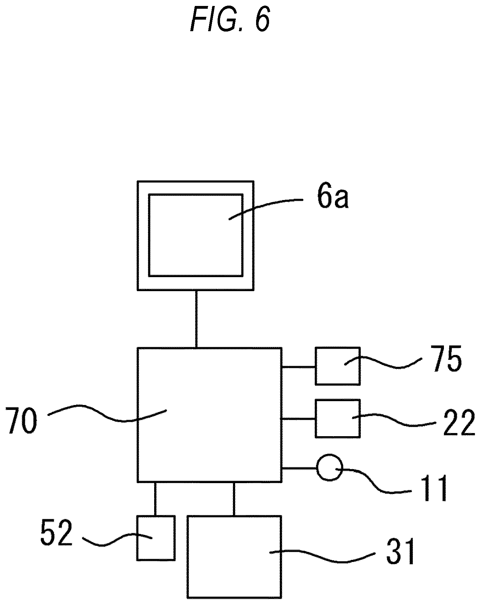

FIG. 6 is a view showing a control system of the optometry device 100. For example, various members such as the monitor 6a, a non-volatile memory 75 (hereinafter referred to as a memory 75), the light source 11 included in the measurement unit 7, the imaging element 22, the display 31, the imaging element 52, and the like are electrically connected to the controller 70. Further, for example, the controller 70 is electrically connected to a driving unit that is not shown provided with the driving unit 9, the drive mechanism 39, the driving unit 83, and the like.

For example, the controller 70 includes a CPU (processor), a RAM, a ROM, and the like. For example, the CPU controls each member of the optometry device 100. For example, the RAM temporarily stores various pieces of information. For example, various programs for controlling the operation of the optometry device 100, the visual target, initial values, and the like are stored in the ROM. The controller 70 may include a plurality of control units (that is, a plurality of processors).

For example, the memory 75 is a non-transitory storage medium that can hold stored contents even in a case where power supply is interrupted. For example, a hard disk drive, a flash ROM, a USB memory, or the like can be used as the memory 75. For example, the memory 75 stores a control program for controlling the objective measurement unit and the subjective measurement unit.

### <Control operation>

The control operation of the optometry device 100 will be described.

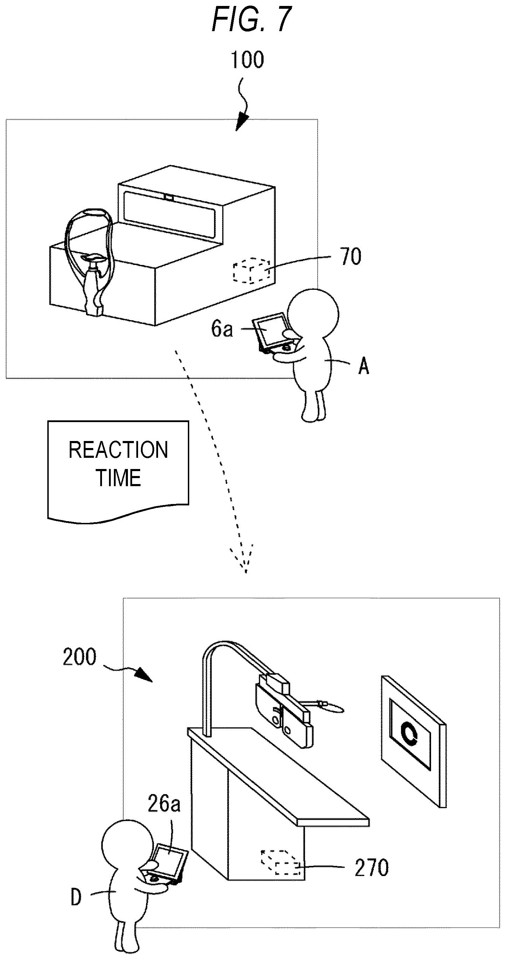

FIG. 7 is a view showing an example of a flow of the subjective examination on the subject eye E. Here, a case where a subj ective examination proceeds using two devices, a first subjective optometry device (optometry device 100) and a second subjective optometry device 200 (hereinafter referred to as an optometry device 200), is exemplified. It is needless to say that the subjective examination may proceed using one device, or may proceed using two or more devices.

First, in the subjective examination (subjective measurement), an examination (measurement) with respect to the subject eye E is performed using the optometry device 100 by an examinee or an assistant A who assists an examiner D. For example, the subject eye E is subjected to at least one examination of the naked eye visual acuity examination, refractive correction examination, or the like. The assistant A may be unfamiliar with examinations.

For example, the assistant A sets at least one of a predetermined visual target to be presented to the subject eye E or a predetermined correction power for correcting the subject eye E, and starts the examination. In addition, for example, the assistant A asks the examinee whether the visual target can be confirmed, and obtains a response from the examinee. For example, the controller 70 of the optometry device 100 acquires the reaction time required from the start of the examination until the response of the examinee is obtained. For example, the assistant A switches at least one of the visual target or the correction power in consideration of the content and correctness of the response of the examinee, starts the examination again, and obtains a response from the examinee. For example, the controller 70 acquires the reaction time again. For example, the reaction time of the examinee is acquired sequentially by repeating such an operation by the assistant A. For example, the controller 70 causes the monitor 6a included in the optometry device 100 to display the acquired reaction time.

Subsequently, in the subjective examination, the examiner D who is familiar with the examination performs an examination with respect to the subject eye E, using the optometry device 200 different from the optometry device 100. For example, the examiner D may check the monitor 6a of the optometry device 100, determine whether or not the examination result of the subject eye E is appropriate based on the reaction time of the examinee, and perform reexamination as necessary. As an example, based on the reaction time of the examinee, only the state where a predetermined visual target and a predetermined correction power are set may be reexamined. As a result, the examiner can accurately obtain correct examination results of the subject eye E. In addition, accordingly, the examiner can efficiently proceed with the examination of the subject eye E.

Note that such reaction time of the examinee may be transmitted from the optometry device 100 to the optometry device 200. For example, a controller 270 of the optometry device 200 may receive the reaction time of the examinee and cause the monitor 26a included in the optometry device 200 to display the reaction time. For example, the examiner D may check the monitor 26a of the optometry device 200 to determine whether or not to perform the reexamination.

In the following, a more detailed description will be given by taking as an example a case where the subject eye E is subjected to a refractive correction examination.

### <Alignment between subject eye and measurement unit>

First, alignment between the subject eye E and the measurement unit 7 is performed. For example, in a case where the assistant A selects an alignment start switch, which is not shown, of the switch unit 6b, the controller 70 moves the measurement unit 7 with respect to the subject eye E. As a result, the subject eye E and the optical axis L2 in the subjective measurement optical system 25 match (substantially match) each other.

### <Acquisition of reference value for reaction time>

Next, a preliminary measurement with respect to the subject eye E is started, and a reference value of the reaction time of the examinee is set. For example, since there are individual differences in the reaction speed of examinees, the timing at which the examinees can respond immediately after correctly recognizing a visual target differs. In other words, the reaction time required for the examinee to immediately respond after correctly recognizing the visual target changes for each examinee.

Therefore, the controller 70 controls the display of the monitor 6a to display a visual target that can be easily recognized by the examinee. For example, a visual target that can be easily recognized by the examinee may be a visual target that allows the examinee to immediately give a correct answer. In other words, any visual target may be used as long as the examinee can respond with confidence. In addition, the controller 70 acquires the reaction time from the time when the examinee recognizes such a visual target to the time when the examinee responds, and sets the reaction time as a reference value.

As an example, in a case where the reaction speed of the examinee is fast and the response is obtained in 1 second after the visual target is displayed on the monitor 6a (after the visual target is presented to the subject eye), the controller 70 sets the reference value of the reaction time of the examinee to be 1 second. Further, as an example, in a case where the reaction speed of the examinee is slow and the response is obtained in 3 seconds after the visual target is displayed on the monitor 6a (after the visual target is presented to the subject eye), the controller 70 sets the reference value of the reaction time of the examinee to be 3 seconds. For example, by presetting a reference value of the reaction time for each examinee, it is possible to accurately perform a refractive correction examination that will be described later.

For example, the assistant A operates the switch unit 6b to select a predetermined visual target. As an example, the assistant A selects the Landolt ring visual target having the lowest visual acuity value. For example, the controller 70 controls the display 31, according to the selection signal from the switch unit 6b, to display the Landolt ring visual target. Further, for example, the controller 70 causes the memory 75 to store the timing at which the Landolt ring visual target is displayed on the display 31.

For example, in a case where the assistant A asks the examinee about the direction of the gap in the Landolt ring visual target and the examinee responds, the assistant A operates the switch unit 6b to select whether the response of the examinee is correct or incorrect. In a case where the assistant A selects whether the response of the examinee is correct or incorrect, a response signal representing the response of the examinee is input. The controller 70 causes the memory 75 to store the response timing at which the examinee responds, based on the response signal.

The controller 70 acquires the reaction time from the time when the display 31 is controlled to the time when the response signal is obtained, based on the start timing and the response timing. In other words, the reaction time from time when the visual target is presented to the subject eye E to the time when the response of the examinee is obtained is acquired, based on the start timing and the response timing. For example, the controller 70 acquires the difference between the start timing and the response timing, as the reaction time. Further, for example, the controller 70 causes the memory 75 to store such a reaction time of the examinee, as a reference value.

The assistant A may perform the same operation plural times by changing the direction of the gap of the Landolt ring visual target to be presented to the subject eye E. For example, in this case, the controller 70 may store the average reaction time of the examinee in the memory 75, as a reference value.

### <Start of refractive correction examination>

Next, measurement of the subjective eye refractive power (subjective value) of the examinee is started. The assistant A operates the switch unit 6b to select a predetermined correction power (first correction power). For example, the assistant A may select the spherical power, the cylindrical power, the astigmatism axis angle, and the like for correcting the subject eye E, based on the objective eye refractive power (objective value) of the subject eye E acquired in advance. As an example, in the present example, the objective values of the subject eye E are obtained as a spherical power of -3D, a cylindrical power of 0D, and an astigmatism axis angle of 0 degrees, and the assistant A selects -3D as the spherical power, 0D as the cylindrical power, and 0 degrees as the astigmatism axis angle for correcting the subject eye E. That is, the assistant A selects each value to correct the subject eye E at 0D.

The controller 70 controls at least one of the light projecting optical system 30 or the correction optical system 60, according to the selection signal from the switch unit 6b. For example, the controller 70 may correct the spherical power of the subject eye E by driving the drive mechanism 39 and moving the display 31 in the optical axis L2 direction. Further, for example, by driving the rotation mechanism 62a and the rotation mechanism 62b to rotate the cylindrical lens 61a and the cylindrical lens 61b around the optical axis L2b, the controller 70 may correct at least one of the cylindrical power or the astigmatism axis angle of the subject eye E. As a result, the subject eye E is corrected with the first correction power.

### <Acquisition of reaction time in refractive correction examination>

After correcting the subject eye E with the first correction power, the assistant A operates the switch unit 6b to select a predetermined visual target. For example, the assistant A may select each of the visual acuity value of the visual target and the type of the visual target. As an example, in the present example, a Landolt ring visual target with a visual acuity value of 1.0 may be selected.

The controller 70 controls the display 31, according to the selection signal from the switch unit 6b, to display the Landolt ring visual target on the screen thereof. In addition, the controller 70 also causes the memory 75 to store the timing at which the Landolt ring visual target is displayed on the display 31 (that is, the start timing at which the examination of the examinee eye is started).

The assistant A asks the examinee about the direction of the gap of the Landolt ring visual target in a state where the subject eye E is corrected with the first correction power, and obtains a response from the examinee. Further, in a case where the assistant A obtains the response from the examinee, the assistant A operates the switch unit 6b to select whether the response of the examinee is correct or incorrect. For example, in a case where the assistant A selects whether the response of the examinee is correct or incorrect, a response signal representing the response of the examinee is input. Based on the response signal from the switch unit 6b, the controller 70 causes the memory 75 to store the timing at which whether the response of the examinee is correct or incorrect is selected (that is, the response timing at which the examinee responds).

The controller 70 acquires the reaction time required from the start of the examination to the time when the response of the examinee is obtained, based on the start timing at which the examination of the subject eye is started and the response timing at which the examinee responds. More specifically, the subject eye E is corrected with the first correction power (spherical power of -3D, cylindrical power of 0D, and astigmatism axis angle of 0 degrees), , and the reaction time required, from the start of the presentation of the Landolt ring visual target with a visual acuity value of 1.0, to the time when the response of the examinee is obtained is acquired. For example, the controller 70 acquires the reaction time from the difference between the start timing and the response timing. As a result, the reaction time of the subject eye E with respect to the first correction power is obtained.

The assistant A may change the direction of the gap of the Landolt ring visual target with a visual acuity value of 1.0 presented to the subject eye E, and obtain the response of the examinee each time. Based on the selection signal from the switch unit 6b, the controller 70 causes the memory 75 to store the start timing each time the display 31 is controlled to change the direction of the first visual target (each time the examination of the subject eye is started). Further, the controller 70 causes the memory 75 to store the response timing each time the response signal of the examinee is input (each time the examinee responds), based on the selection signal from the switch unit 6b. Thereby, the controller 70 can acquire a plurality of reaction times in a case where the subject eye E is corrected with the first correction power and the Landolt ring visual target with a visual acuity value of 1.0 is presented. For example, the controller 70 may obtain the average time of the plurality of reaction times, as the reaction time of the subject eye E, with respect to the first correction power.

For example, the assistant A confirms whether or not the first correction power of the subject eye E is appropriate, and in a case where the first correction power is confirmed to be inappropriate, the assistant A corrects the subject eye E with a second correction power different from the first correction power. As an example, the assistant A may select a spherical power of -2.75D, a cylindrical power of 0D, and an astigmatism axis angle of 0 degrees. That is, the assistant A may select each value to correct the subject eye E by -0.25D. The controller 70 controls at least one of the light projecting optical system 30 or the correction optical system 60 according to the selection of the switch unit 6b by the assistant A, changes the first correction power to the second correction power, and causes the memory 75 to store the start timing when the second correction power is set. Further, the controller 70 causes the memory 75 to store the response timing at which the response is obtained in a case where the subject eye E is corrected with the second correction power, according to the selection of the switch unit 6b by the assistant A. For example, the controller 70 acquires the reaction time of the subject eye E with respect to the second correction power based on the difference between the start timing and the response timing. It is needless to say that the average time of a plurality of reaction times obtained each time the direction of the gap of the Landolt ring visual target is changed may be obtained as the reaction time of the subject eye E with respect to the second correction power.

For example, during the refractive correction examination of the subject eye E, as described above, the assistant A switches at least one of the correction power of the subject eye E or the orientation of the gap of the Landolt ring visual target with a visual acuity value of 1.0, in consideration of the response of the examinee. For example, the correction power of the subject eye E, which is determined to be appropriate by the assistant A, is acquired as the subjective value of the subject eye E.

For example, the controller 70 obtains the reaction time of the examinee sequentially based on the start timing at which any one of the display 31, the light projecting optical system 30, and the correction optical system 60 is controlled to switch the visual target and the correction power, and the response timing at which the examinee responds with respect to this switching, during the refractive correction examination of the subject eye E. Further, for example, the controller 70 may obtain the reaction time required for each examination item by adding the reaction times of the examinee obtained each time when the correction power of the subject eye E and the direction of the gap of the Landolt ring visual target with a visual acuity value of 1.0 are switched, during the refractive correction examination of the subject eye E.

### <Display of reaction time>

For example, when acquiring the reaction time of the examinee, the controller 70 cause the monitor 6a to display the reaction time. For example, in a case where the refractive correction examinations are sequentially performed with respect to one eye, the reaction time for one eye measurement may be displayed. Further, for example, in a case where the refractive correction examinations are performed with respect to both eyes, the reaction time for both eye measurement may be displayed. It is needless to say that, in a case where the refractive correction examination is performed with respect to one eye and both eyes, at least one of the reaction time for the one eye measurement or the reaction time for the both eye measurement may be displayed.

For example, the controller 70 may cause the monitor 6a to display reaction times with respect to different start timings during a refractive correction examination such that the reaction times can be compared. As an example, a graph (graph 500 to be described later) showing the reaction time of the examinee required for each examination item in the refractive correction examination may be generated and displayed on the monitor 6a. Further, as an example, a graph (graph 600 described later) showing the relationship between the reaction time of the examinee and the correction power for correcting the subject eye E may be generated and displayed on the monitor 6a. It is needless to say that, for example, the controller 70 may cause the monitor 6a to display at least one of the reaction time of the examinee, the examination time required for the refractive correction examination, the subjective value of the subject eye E, the number of times (frequency) of switching the correction power, or the like.

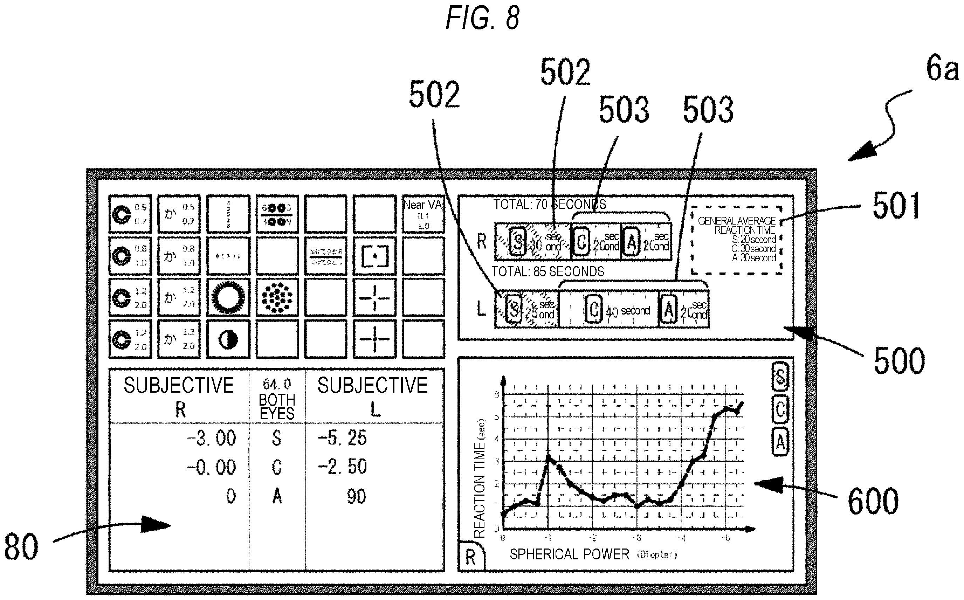

FIG. 8 is an example of the monitor 6a. For example, the monitor 6a displays a result image 80 showing the examination result of the subject eye E, the graph 500 showing the reaction time required for each examination item in the refractive correction examination, the graph 600 showing the reaction time and the correction power in the refractive correction examination, and the like.

For example, in the present example, a bar graph is taken as an example of the graph 500, but the graph 500 may be a bar graph, a broken line graph, an area graph, an approximate curve graph, or any other type of graph. Further, for example, in the present example, the graph 500 may display a general average reaction time 501 required for each examination item.

For example, the graph 500 includes a reaction time 502 of a spherical examination, in which the reaction times obtained each time the spherical power for correcting the subject eye E is switched are combined in the spherical examination of the refractive correction examination. Further, for example, the graph 500 includes a reaction time 503 of an astigmatism examination in which the reaction times obtained each time the cylindrical power or the astigmatism axis angle for correcting the subject eye E is switched are combined, in the astigmatism examination of the refractive correction examination. The reaction time 503 of the astigmatism examination may be divided into a reaction time for switching the cylindrical power and a reaction time for switching the astigmatism axis angle.

For example, the examiner can easily understand the item that took a long time to respond by confirming the reaction time of each examination item with respect to the examinee from the graph 500. As an example, the examiner may determine whether the subj ective value of the subject eye E is reliable from the reaction time of each examination item. In addition, as an example, in a case where the reaction time for an examination item is extremely short or extremely long with respect to the general average reaction time 501, the examiner may perform examination again with respect to the examination item.

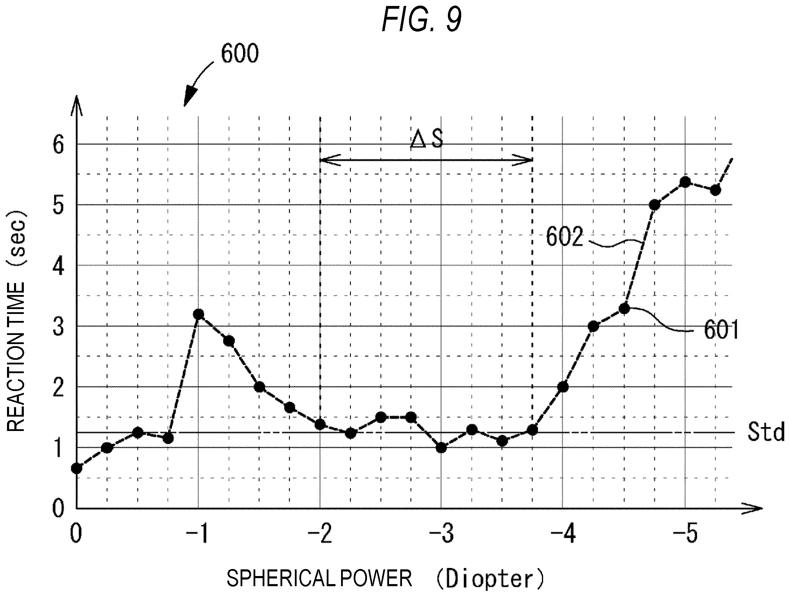

FIG. 9 is an example of the graph 600 showing the relationship between the reaction time and the correction power in the refractive correction examination. In the present example, a broken line graph is taken as an example of the graph 600, but the graph 600 may be a broken line graph, a bar graph, an area graph, an approximate curve graph, or any other type of graph. For example, the vertical axis of the graph 600 is the reaction time of the examinee, and the horizontal axis is the correction power (spherical power in the present example) for correcting the subject eye E.

For example, the graph 600 includes a plurality of points 601 where the reaction time of the examinee is plotted for each spherical power. That is, the graph 600 includes a plurality of plotted points 601 where the reaction time of the examinee is plotted for each time the spherical power for correcting the subject eye E is switched. In addition, for example, the graph 600 includes a broken line 602 generated based on the plurality of plotted points 601. The graph 600 may include either the plurality of plotted points 601 or the broken lines 602. Further, for example, the graph 600 may include a reference line Std that is generated based on the reference value of the reaction time of the examinee.

For example, by checking the reaction time of the examinee using the graph 600, the examiner can estimate various pieces of information such as whether the examinee gives an answer intuitively, whether the examinee easily recognizes the visual target, or whether the subject eye E is in an adjustment state.

As an example, in a case where the subject eye E can clearly visually recognize the Landolt ring visual target and the examinee gives an answer with confidence, the examinee gives an answer at a predetermined reaction time. However, in a case where the subject eye E cannot visually recognize the Landolt ring visual target and the examinee answers "I don't recognize it" or answers by feeling (intuition), the reaction time of the examinee is approximately the same as the predetermined reaction time, or tends to be shorter than the predetermined reaction time. Therefore, the examiner can estimate the correction power at which the examinee may give an answer by feeling (or the range of correction power at which the examinee may give an answer by feeling), from the change in the plotted point 601.

Further, as an example, in a case where the correction power for correcting the subject eye E is appropriate, the subject eye E can clearly visually recognize the Landolt ring visual target, and the reaction time of the examinee tends to be shortened. However, in a case where the correction power for correcting the subject eye E is inappropriate, the subject eye E becomes foggy and the Landolt ring visual target is blurred to be visually recognized, and thus, the reaction time of the examinee tends to be lengthened. Therefore, the examiner can estimate the correction power appropriate for the subject eye E, from the change in the plotted points 601.

Further, as an example, in a case where the adjustment of the subject eye E works, the subject eye E can focus on the Landolt ring visual target, and thus the Landolt ring visual target can be clearly visually recognized, and the reaction time of the examinee tends to be shortened. However, in a case where the adjustment of the subject eye E does not work (or in a case where the adjustment of the subject eye E is released), the subject eye E cannot focus on the Landolt ring visual target, the Landolt ring visual target is blurred to be visually recognized, and thus, the reaction time of the examinee tends to be lengthened. Therefore, the examiner can estimate a range ΔS of the correction power (spherical power) at which the adjustment of the subject eye E works, from the change in the plotted points 601.

By comparing the reference line Std based on the reaction time in a case where the visual target that can be responded by the examinee with confidence is presented with each plotted point 601, it becomes easier for the examiner to determine the probability that the examinee gives an answer by feeling, the appropriate correction power, the range ΔS in which adjustment works, and the like. For example, the examiner may examine again only the correction power (spherical power) at which the reaction time of the examinee is shortened.

In a case where the answer of the examinee in the refractive correction examination is "I don't recognize it" or is an incorrect answer, a display may be made on the monitor 6a such that the examiner can understand that fact. For example, the marker (for example, circle, square, triangle, and the like) of the plotted point 601 corresponding to each correction power may be changed according to the answer of the examinee. Further, for example, a symbol (for example, "?", "x", and the like) may be indicated along with the plotted points 601 corresponding to each correction power according to the answer of the examinee. It is needless to say that, even in a case where the answer of the examinee is correct, similarly, this may be displayed on the monitor 6a.

In the present example, a configuration in which the assistant operates the switch unit 6b to input the response of the examinee has been described as an example, but the present invention is not limited thereto. For example, a configuration in which the examinee himself or herself inputs a response may be adopted. In this case, the optometry device 100 may include an examinee controller for the examinee to operate. As an example, the examinee controller may have a response lever that can be tilted according to the direction of the gap of the Landolt ring visual target, and a button that is pressed in a case where the Landolt ring visual target cannot be recognized. The controller 70 may cause the memory 75 to store the response timing based on the response signal input from at least one of the response lever or the button.

In the present example, the case where the reference value of the reaction time of the examinee is acquired in the preliminary measurement for the subject eye E has been described as an example, but the present invention is not limited thereto. For example, it is not necessary to acquire the reference value of the reaction time of the examinee. In this case, the inclination of the broken line 602 or the like may be used to estimate an appropriate correction power or the like for the subject eye E.

Moreover, in the present example, a configuration in which the subject eye E is placed in the naked eye state in the preliminary measurement for the subject eye E has been described as an example, but the present invention is not limited thereto. For example, the subject eye E may be in a correction state. In this case, the correction power for correcting the subject eye E may be selected based on the objective value of the subject eye.

### <Second example>

A second example of the optometry system according to the present embodiment will be described. Here, a case where the optometry device 100 is configured to be used for self-optometry is taken as an example. In the second example, configurations similar to those in the first example (for example, members, optical systems, measurement optical paths, internal configurations, and the like) will be given the same reference numerals as in the first example, and the detailed descriptions thereof will be omitted.

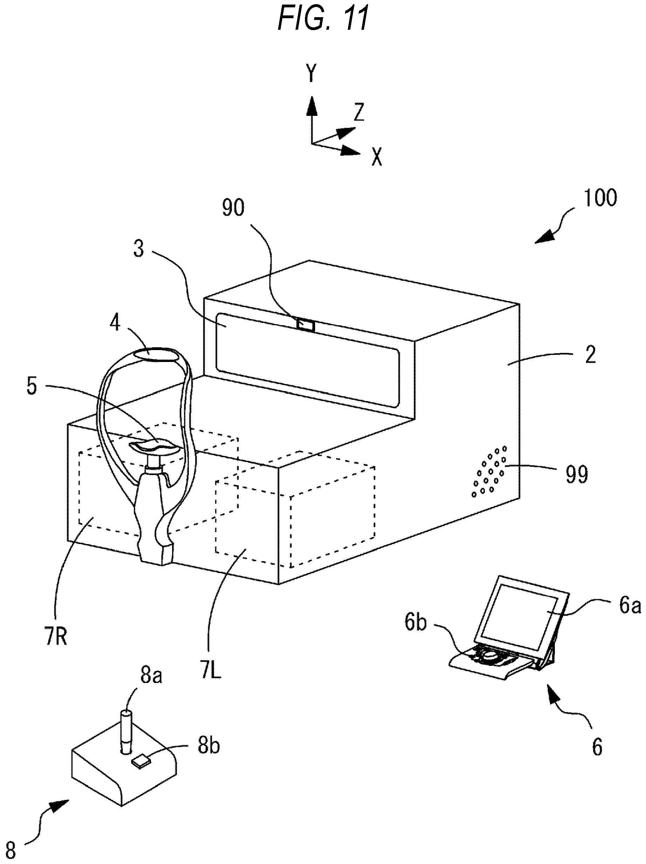

FIG. 11 is an example of the optometry device 100. The optometry device 100 may further include a speaker 99, the examinee controller 8, and the like in addition to the configuration of the first example. The speaker 99 outputs a voice guide or the like. The left and right speakers 99 may be provided one by one, or one speaker serve as the left and right speakers.

The examinee controller 8 is used when inputting the reading results obtained by reading the examination visual target presented by the examinee. The examinee controller 8 includes an answer lever 8a, an answer button 8b, and the like. For example, the answer lever 8a can input signals in four directions, up, down, left, and right, corresponding to the directions of the cuts of the Landolt ring visual target, by a tilting operation. For example, the answer button 8b can be pressed to input a signal indicating a case where the examination visual target is unreadable (illegible). A signal from the examinee controller 8 is output to the controller 70 through wired communication or wireless communication.

### <Control operation>

The control operation of the optometry device 100 will be described.

In the self-optometry, after alignment between the subject eye E and the measurement unit 7 is performed, a reference value of the reaction time of the examinee is set in a preliminary measurement with respect to the subject eye E. At this time, for example, the controller 70 may acquire the reference value based on the timing at which the visual target is displayed on the display 31 and the timing at which the examinee controller 8 is operated, and set the value.

For example, the controller 70 causes the display 31 to display the Landolt ring visual target that can be easily recognized by the examinee, and causes the memory 75 to store the timing of displaying this visual target. Further, a voice guide is generated from the speaker 99 to ask the examinee the direction of the cuts in the Landolt ring visual target. For example, the examinee reads the direction of the cuts in the Landolt ring visual target and tilts the answer lever 8a in the direction of the cuts. For example, the controller 70 causes the memory 75 to store the timing at which the response signal input along with the tilting of the answer lever 8a is acquired.

The controller 70 may acquire the difference between these two timings as a reference value. It is needless to say that the reference value may be an average obtained by obtaining the difference between these two timings a plurality of times.

In a case where the reference value of the reaction time of the examinee is obtained, an objective examination and a subjective examination with respect to the subject eye E are sequentially performed. For example, the objective value of the subject eye E is obtained by an objective examination. Further, for example, in a state where the subject eye E is corrected to a predetermined eye refractive power (such as 0D) with the first correction power (that is, the state where the first correction power is used as the initial value) based on the objective value of the subject eye E, the subjective examination of the subject eye E is started. Here, similarly to the first example, the refractive correction examination is started.

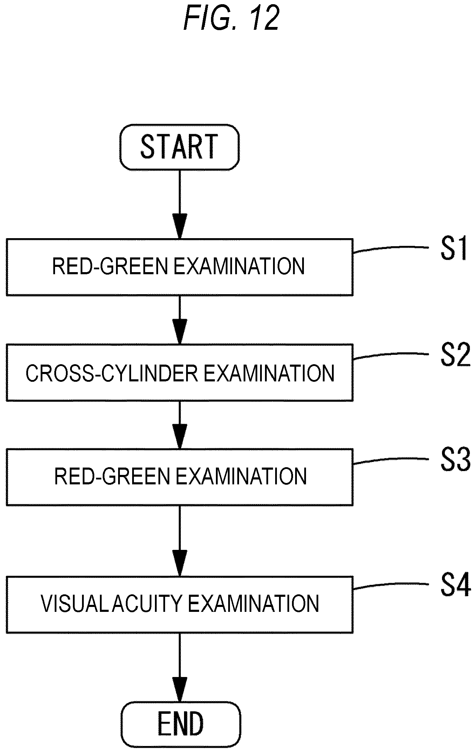

FIG. 12 is a flowchart showing the detailed flow of the refractive correction examination. For example, the refractive correction examination includes a plurality of examination items, and the optometry for each examination item is automatically proceeded by executing the self-optometry application. As an example, in the present example, a red-green examination and a cross-cylinder examination (steps S1 to S3) for adjusting the first correction power for correcting the subject eye E to an appropriate power (second correction power), and a visual acuity examination (step S4) for measuring the highest visual acuity value in the state where the subject eye E is corrected with the second correction power, are sequentially performed.

In the refractive correction examination of the subject eye E, especially in self-optometry in which the examiner or the assistant is not present near the examinee, the state where the examinee finds himself or herself in trouble during the examination may occur. As an example, there is a state where the examinee forgets the operation method of the examinee controller 8, or the like. Therefore, the controller 70 appropriately outputs leading information for leading the examinee to input an answer, based on the reference value of the reaction time of the examinee. In the present example, a voice guide for conveying the operation method of the examinee controller 8 is generated from the speaker 99, as leading information. Details of this will be described later.

### <Red-green examination (S1)>

First, a red-green examination is performed in order to adjust the first spherical power of the first correction power for correcting the subject eye E. In the red-green examination, it is possible to determine which of near-sighted, emmetropic, and far-sighted states corresponds to the state where the subject eye is corrected with a desired spherical power. For example, the near-sighted state is determined in a case where the clearly visually recognized visual target is red, the far-sighted state is determined in a case where the clearly visually recognized visual target is green, and the emmetropic state is determined in a case where red and green are visually recognized at the same level. For example, the far-sighted state is overcorrected and the spherical power is lowered until a state becomes the emmetropic state (or near-sighted state).

At the start of the red-green examination, the controller 70 causes the speaker 99 to generate a voice guide indicating the operation method of the examinee controller 8 in the red-green examination. As an example, the voice guide may be generated, in which the voice guide indicate that the answer lever 8a is tilted to the left in a case where the clearly visually recognized visual target, among the red visual target and the green visual target, is red, the answer lever 8a is tilted to the right in a case where the clearly visually recognized visual target, among the red visual target and the green visual target, is green, and the answer button 8b is pressed in a case where the red visual target and the green visual target are visually recognized at the same level.

Next, the controller 70 proceeds with the examination and causes the display 31 to display the red-green visual target. For example, a red-green visual target is displayed with red on the left and green on the right. In addition, the controller 70 generates voice guide asking the examinee about the color of the visual target that can be clearly visually recognized. The examinee confirms the visual target and tilts the answer lever 8a to the left or right, or presses the answer button 8b.

In a case where the controller 70 acquires a response signal from the answer lever 8a obtained by selecting either red or green visual target by the examinee and, the controller 70 increases or decreases the spherical power by one step according to the tilting direction of the answer lever 8a. That is, the drive mechanism 39 is driven to move the display 31 in the optical axis L2 direction. In addition, the controller 70 again generates the voice guide asking about the color of the visual target. For example, the controller 70 repeats such control to automatically proceed with the examination.

The controller 70 ends the red-green examination in a case where a response signal from the answer button 8b by the examinee determining that the red and green visual targets are at the same level is obtained. The spherical power at which the subject eye E has been corrected at the end of the examination is taken as the spherical power (second spherical power) at the start of the visual acuity examination.

### <Cross-cylinder examination (S2)>

Next, a cross-cylinder examination is performed in order to adjust at least one of the first astigmatism axis angle or the first cylindrical power among the first correction powers for correcting the subject eye E. In the cross-cylinder examination, it can be determined whether the astigmatism axis angle of the subject eye matches the desired astigmatism axis angle for correcting the subject eye. For example, in a case where there is a difference in the appearance of the two point group visual targets, it is determined that each angle does not match each other, and in a case where the appearances of the two point group visual targets are approximately the same, it is determined that each angle matches each other. Further, in the cross-cylinder examination, it is possible to determine whether the state where the subject eye is corrected with a desired cylindrical power is appropriate. For example, in a case where there is a difference in appearance of the two point group visual targets, it is determined that the state is determined to be inappropriate, and in a case where appearances of the two point group visual targets are approximately the same, it is determined that the state is determined to be appropriate.

At the start of the cross-cylinder examination, the controller 70 causes the speaker 99 to generate a voice guide indicating the operation method of the examinee controller 8 in the cross-cylinder examination. As an example, the voice guide indicating that the answer lever 8a is tilted to the left in a case where the clearly visually recognized visual target, among the two point group visual targets, is the left visual target, the answer lever 8a is tilted to the right in a case where the clearly visually recognized visual target, among the two point group visual targets, is the right visual target, and the answer button 8b is pressed in a case where the left visual target and the right visual target are visually recognized at the same level.

The controller 70 proceeds with the examination, and first adjusts the astigmatism axis angle. The controller 70 sets the cylindrical lens 61a and the cylindrical lens 61b to the axis angles for adjusting the astigmatism axis angle. In addition, the controller 70 cause the display 31 to display the point group visual target, and generate a voice guide to ask the position of the visual target that can be clearly visually recognized by the examinee. In a case where a response signal from the answer lever 8a is obtained (in a case where the position of the visual target is selected), the controller 70 changes the astigmatism axis angle according to the tilting direction of the answer lever 8a. That is, the rotation mechanism 62a and the rotation mechanism 62b are driven to rotate the cylindrical lens 61a and the cylindrical lens 61b. In addition, the controller 70 again generates the voice guide asking about the position of the visual target. For example, the controller 70 repeats such control until a response signal is obtained from the answer button 8b (until the answer that the appearances of the two visual targets are approximately the same is received) to automatically proceed with the examination.

In a case where a response signal from the answer button 8b is obtained, the controller 70 switches from adjusting the astigmatism axis angle to adjusting the cylindrical power. The astigmatism axis angle at which the subject eye E is corrected at this time is taken as the astigmatism axis angle (second astigmatism axis angle) at the start of the visual acuity examination.

The controller 70 then adjusts the cylindrical power. The controller 70 sets the cylindrical lens 61a and the cylindrical lens 61b at axis angles for adjusting the cylindrical power, and generates a voice guide asking about the position of the visual target. In a case where a response signal from the answer lever 8a is obtained, the controller 70 increases or decreases the cylindrical power by one step according to the tilting direction of the answer lever 8a, and again generates a voice guide asking about the position of the visual target. For example, the controller 70 repeats such control until a response signal is obtained from the answer button 8b to automatically proceed with the examination.

In a case where a response signal from the answer button 8b is obtained, the controller 70 ends the adjustment of the cylindrical power. The cylindrical power corrected for the subject eye E at this time is taken as the cylindrical power (second cylindrical power) at the start of the visual acuity examination. Further, the controller 70 ends the cross-cylinder examination.

### <Red-green examination (S3)>

The controller 70 may perform the red-green examination again in order to adjust the second spherical power of the subject eye E. For example, here, it is confirmed whether or not the adjustment of the subject eye works. As an example, at the end of step S1 and at the start of step S3, the correction is made with the same spherical power, but in a case where the adjustment works, the appearance of the red-green visual target changes. The red-green examination in step S3 is basically the same as the red-green examination in step S1, and thus the description thereof will be omitted.

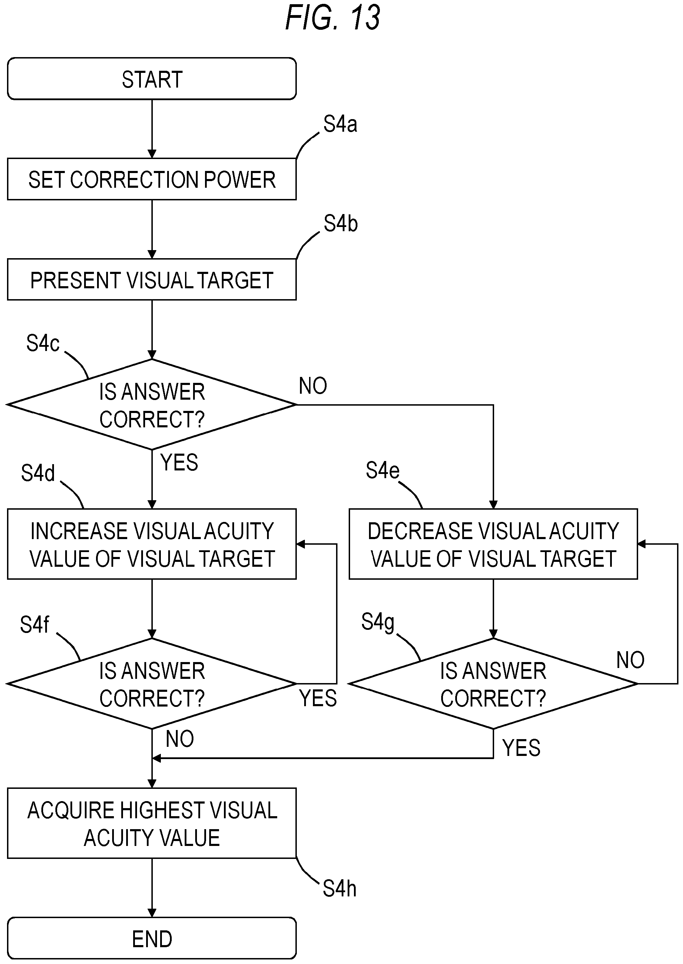

The first correction power for correcting the subject eye E is changed to the second correction power by adjusting the spherical power in the red-green examination in steps S 1 and S3, and adjusting the astigmatism axis angle and the cylindrical power in the cross-cylinder examination in step S2. That is, the subject eye E is corrected with the second spherical power, the second astigmatism axis angle, and the second cylindrical power (step S4a in FIG. 13).

### <Visual acuity examination (S4)>

FIG. 13 is an example of the flow of the visual acuity examination. In a state where the subject eye E is corrected with the second correction power, a visual acuity examination is performed to acquire the highest visual acuity value.

At the start of the visual acuity examination, the controller 70 causes the speaker 99 to generate a voice guide indicating the operation method of the examinee controller 8 in the visual acuity examination. As an example, a voice guide for tilting the answer lever 8a in accordance with the direction of the cuts in the Landolt ring visual target, and pressing the answer button 8b when the direction of the cuts is unknown, may be generated.

The controller 70 proceeds the examination and causes the display 31 to display the Landolt ring visual target of a predetermined visual acuity value (step S4b). As an example, a Landolt ring visual target with a visual acuity value of 1.0 is displayed. In addition, the controller 70 generates a voice guide asking about the direction of the cuts in the Landolt ring visual target.

In a case where a response signal from the answer lever 8a is received, the controller 70 detects whether the response of the examinee is correct or incorrect (step S4c). In a case where the answer of the examinee is correct, the Landolt ring visual target is switched to the Landolt ring visual target with one step higher visual acuity value (for example, the Landolt ring visual target with a visual acuity value of 1.2) (step S4d), and the voice guide asking about the direction of the cuts is generated again. In a case where the answer of the examinee is incorrect, the Landolt ring visual target is switched to the Landolt ring visual target with one step lower visual acuity value (for example, the Landolt ring visual target with a visual acuity value of 0.9) (step S4e), and the voice guide asking about the direction of the cuts is generated again. For example, in a case where a response signal is received from the answer button 8b, the answer of the examinee may be regarded as equivalent to an incorrect answer, and the processing may proceed to step S4e.

In a case where the processing proceeds to step S4d, the controller 70 detects whether the answer of the examinee is correct or incorrect (step S4f). In a case where the answer of the examinee is correct, the processing returns to step S4d to further increase the visual acuity value of the Landolt ring visual target. In a case where the answer of the examinee is incorrect, the highest visual acuity value in the state where the subject eye E is corrected with the second correction power is obtained (step S4h). For example, at this time, instead of the visual acuity value of the Landolt ring visual target presented to the subject eye E (incorrectly answered Landolt ring visual target), the visual acuity value of the correctly answered Landolt ring visual target presented in the previous state, is obtained as the highest visual acuity value.

Further, in a case where the processing proceeds to step S4e, the controller 70 detects whether the answer of the examinee is correct or incorrect (step S4g). In a case where the answer of the examinee is incorrect, the processing returns to step S4e to further decrease the visual acuity value of the Landolt ring visual target. In a case where the answer of the examinee is correct, the highest visual acuity value in the state where the subject eye E is corrected with the second correction power is obtained (step S4h). For example, at this time, the visual acuity value of the Landolt ring visual target presented to the subject eye E (correctly answered Landolt ring visual target) is obtained as the highest visual acuity value.

In the refractive correction examination, a value different from the highest visual acuity value of the subject eye may be acquired. For example, the value of the most positive correction power (that is, the completely corrected value) that provides the highest visual acuity of the subject eye E may be acquired.

### <Output of leading information>

There is a possibility that the examinee will forget the operation method of the examinee controller 8 during the process of each examination item in the refractive correction examination. For example, in the red-green examination, in a case where the appearances of each visual targets are approximately the same after the state of selecting either red or green visual target continues, there is a case where the examinee forget what to do. Similarly, for example, in the cross-cylinder examination or the visual acuity examination, in a case where the appearance of the visual target changes, or the like, there is a case where the examinee forget what to do. Since the operation method of the examinee controller 8 differs for each of the plurality of examination items, the examinee may be confused. For example, in a case where the examinee operates the answer lever 8a or the like inappropriately for this reason, the examination will automatically proceed, which may affect the examination results or cause rework of the examination that takes time.

Therefore, the controller 70 measures the time from the start timing to the response timing each time, even in self-optometry. In other words, the reaction time of the examinee is acquired sequentially. For example, the start timing is the timing at which the visual target is displayed and the timing at which the correction power (at least one of the spherical power, the cylindrical power, or the astigmatism axis angle) is changed. For example, the response timing is the timing at which a response signal is obtained by operating the examinee controller 8.

In addition, the controller 70 considers that the examinee is in trouble in a case where the response signal is not obtained even when a predetermined time based on the reference value of the reaction time of the examinee has elapsed from these start timings, and generates a voice guide indicating the operation method of the examinee controller 8. Such a predetermined time may be a time that does not impose a burden on the examinee. For example, the predetermined time may be the same as the reference value, or the time exceeding the reference value. The time exceeding the reference value may be preset from experiments, simulations, and the like. For example, the time after 3 seconds have elapsed in addition to the reference value, and the like may be used.

The details of the output of the leading information will be described below, taking the red-green examination in steps S 1 and S3 as an example. Here, a case is exemplified in which the predetermined time based on the reference value of the reaction time of the examinee is set to the same time as the reference value.

The controller 70 causes the memory 75 to store the start timing of displaying the red-green visual target, and starts the time measurement. Alternatively, the memory 75 stores the start timing at which a different spherical power is set (the spherical power is changed) while the red-green visual target is displayed, and starts the time measurement. In a case where a response signal from the answer button 8b is obtained before the time equal to the reference value elapses (when there is a response timing), the controller 70 ends the time measurement. In a case where a response signal from the answer button 8b is not obtained even after reaching the time equal to the reference value, a voice guide (first voice guide) indicating the operation method of the examinee controller 8 is generated. The controller 70 automatically proceeds with the examination by, for example, changing the spherical power in a case where a response signal is obtained by the examinee inputting an answer according to the voice guide. For example, in a case where a response signal is obtained while generating the voice guide, the generation of the voice guide may be interrupted and the spherical power may be changed.

In a case where no response signal is obtained from the examinee controller 8 even when a certain time has passed after the generation of the first voice guide, the controller 70 may generate the voice guide (second voice guide) again. For example, in this case, the time is further measured based on the timing at which the first voice guide is generated, and the second voice guide is generated at the timing at which the time equal to the reference value has elapsed after the first voice guide is generated. It is needless to say that the time is not limited to the same time as the reference value, and may be the timing at which a predetermined time obtained by increasing or decreasing the predetermined amount of time from the reference value has elapsed from the reference value, the timing at which a preset time (for example, 5 seconds, and the like) has elapsed, or the like.

For example, such control to repeatedly generate a voice guide may be performed until a response signal from the examinee controller 8 is obtained. Alternatively, in a case where the response signal from the examinee controller 8 is not obtained, the control may be performed until a preset predetermined number of times is reached. In this case, the examination may be forcibly interrupted. The controller 70 may set the time interval for repeating the voice guide to be always the same interval, or may change the interval to gradually shorten the interval or gradually lengthen the time.

Further, the controller 70 may change the content of the voice guide according to the number of times of generating the voice guide. For example, the first voice guide and the second voice guide may differ in content. For example, in the first voice guide, the voice guide may be generated in which the operation method of the examinee controller 8 is associated with the appearance of the visual target of the examinee, as described above. Further, for example, in the second voice guide, the voice guide may be generated in which the operation method of the examinee controller 8 is associated with the call of the examiner. As an example, a voice guide indicating that the answer button 8b is pressed for a long time in order to call the examiner, or the like may be generated.

For example, the content of the first voice guide and the content of the second voice guide may be generated alternately. Further, for example, the content of the second voice guide may be generated after repeating the content of the first voice guide up to a predetermined number of times. In other words, the content of the first voice guide may be switched to the content of the second voice guide in the middle. Accordingly, the examinee can proceed with the examination without hesitation of the examinee in the operation of the examinee controller 8.

It is needless to say that not only the red-green examination but also the cross-cylinder examination and the visual acuity examination may similarly measure the time from the start timing and generate a voice guide based on the reference value. In a case of performing these plural examinations on the subject eye, a reference value of the reaction time of the examinee may be set for each examination item. As a result, it is possible to generate a voice guide at an appropriate timing for each examination item.

In this case, the reference value for the reaction time for each examination item may be set based on the result of the preliminary measurement of the subject eye E. For example, in the preliminary measurement, the reaction time from the time when the examinee recognizes an easily recognizable visual target to the time when the examinee gives an answer may be acquired, and the time obtained by adding a predetermined time different for each examination item to the reaction time may be set as a reference value for the reaction time for each examination item. As an example, the predetermined time different for each examination item may be the time considering the number of visual targets (for example, the number of Landolt ring visual targets displayed at the same time) or the like that the examinee should understand for each examination item.

Further, for example, in the preliminary measurement, the red-green visual target, the cross-cylinder visual target, the Landolt ring visual target, and the like, which are used for each examination item, may be displayed sequentially, and the reaction time from the time when the examinee recognizes such visual targets to the time when the examinee responds may be set as a reference value for the reaction time for each examination item. That is, the reference value for each examination item may be obtained by performing each simple examination in advance.

In the present example, a configuration in which the reaction time obtained in the preliminary measurement for the subject eye is set as the reference value has been described as an example, but the present invention is not limited thereto. For example, during the refractive correction examination, the reaction time obtained appropriately by setting the start timing each time the visual target or the correction power is switched and setting the response timing each time a response signal from the answer button 8b is obtained, may be reflected in the reference value. That is, the reference value for the reaction time may be updated in real time in correspondence with the change in the reaction speed of the examinee as the refractive correction examination proceeds. Accordingly, the reference value for the reaction time of the examinee is optimized.

In this case, for example, the controller 70 may sequentially acquire the reaction time during the refractive correction examination and reflect the average time of a predetermined number of times in the reference value. For example, the predetermined number of times may be preset from experiments or simulations. As an example, an average time of 5 times, or the like may be used. For example, the reaction speed of the examinee tends to increase as the examinee becomes familiar with the examination. Further, for example, the reaction speed of the examinee tends to be slow due to a prolonged examination or an inappropriate correction power for the subject eye. By updating the reference value of the reaction time of the examinee, it is possible to generate a voice guide for leading the examinee to input an answer at a timing at which a burden is not imposed on the examinee.

As described above, for example, the optometry system according to the present example acquires and outputs the reaction time from the start of the examination to the response based on the start timing at which the examination (measurement) of the subject eye is started, which is the start timing at which the visual target presenting means or the correction means is controlled, and the response timing after the start timing, which is the response timing at which the response signal to the examination is input by the response input means. Therefore, the examiner can determine whether or not the examination result of the subject eye is accurate from the reaction time of the examinee. Further, the examiner can easily determine the next setting, and the like from the reaction time of the examinee.

Further, for example, the optometry system according to the present example acquires reaction times at at least two different timings and outputs the reaction times in a comparable manner. Accordingly, it becomes easier for the examiner to compare the reaction times of the examinee and determine the suitability of the examination result of the subject eye. In addition, the examiner can easily determine the operation to be performed next.

Further, for example, the optometry system according to the present example acquires different reaction times at at least two start timings and outputs the reaction times in a comparable manner. For example, accordingly, it becomes easier for the examiner to understand changes in reaction time of the examinee.

Further, for example, the optometry system according to the present example outputs the state of the optometry system together with the reaction time of the examinee. Therefore, the examiner can easily determine whether or not the reaction time of the examinee has increased or decreased according to the change in the state of the optometry system.

Further, for example, the optometry system according to the present example outputs graph data indicating the relationship between the reaction time of the examinee and the optometry system. For example, it is possible to output graph data or the like showing the relationship between the reaction time of the examinee and the correction power set by the correction means. For example, accordingly, the examiner can easily understand the transition in which the reaction time of the examinee changes according to the state of the optometry system.

Further, for example, the optometry system according to the present example sets a reference value based on the reaction time of the examinee with respect to the examination visual target, and based on this reference value, outputs leading information for leading the examinee to input the answer, during the examination of the subject eye. As a result, the hesitation of the examinee in the operation of self-optometry can be reduced, and accurate examination results can be obtained. For example, since the proceeding of the examination caused by the inappropriate operation of the answer lever or the like by the examinee can be reduced, such inappropriate answers are less likely to be reflected in the examination results. In addition, rework of the examination is less likely to occur, and the examination is efficiently performed. In addition, the optometry system can set a different reference value for each examinee, thereby reducing the burden on the examinee and allowing the examination to proceed properly. For example, it is possible to reduce a case where it is determined that the examinee forget the operation method of the answer lever and the leading information is output even though the examinee desires to input an answer. Therefore, the examinee can input answers at his or her own pace, and the examination can proceed comfortably without being rushed or kept waiting.

Further, for example, the optometry system according to the present example repeatedly outputs the leading information, in a case where a signal from the examinee reading the examination visual target and answering is not obtained, even when the predetermined time set for repeating the leading information has elapsed. For example, accordingly, the examinee can easily proceed with the examination even when the examinee forget the operation method of the answer lever or the like during the examination.

Further, for example, the optometry system according to the present example changes and outputs the leading information according to the number of times of output of the leading information. For example, the first leading information is changed to the second leading information that is at least partially different from the first leading information, and then output the leading information. As a result, it is possible to reduce the burden due to repetition of outputs of leading information to the examinee.

Further, for example, the optometry system according to the present example sets reference values based on the reaction time of the examinee according to a plurality of examination items. Therefore, the leading information can be output at appropriate timing for each examination item, and even in a case where the examinee hesitates to answer, the probability of giving an inappropriate answer is reduced.

Further, for example, the optometry system according to the present example acquires the reaction time of the examinee based on the start timing at which the measurement of the subject eye is started and the response timing at which the examinee responds to the examination visual target. Accordingly, it is possible to set the reference value of the reaction time for each examinee in consideration of the reaction speed that differs for each examinee.

Further, for example, the optometry system according to the present example changes and sets the reference value by acquiring the reaction time in real time during the examination. For example, the reference value is changed from the first reference value to the second reference value different from the first reference value and set the reference value. As a result, the reference value based on the reaction time of the examinee is changed each time according to changes in the reaction speed of the examinee, and the leading information is output at appropriate timing. Therefore, the burden on the examinee is reduced. In addition, this leads to shortening of examination time.

### <Modification example>

In the present example, a configuration in which the reference value of the reaction time of the examinee is acquired by performing the preliminary measurement for the subject eye E, has been described as an example, but the present invention is not limited thereto. For example, the reference value of the reaction time of the examinee may be preset according to age or the like. For example, in this case, the examiner may input the examinee information by operating the switch unit 6b. As an example, the examinee information may be an ID assigned to each examinee, and may be the ID associated with a name, age, or the like. For example, the controller 70 may set a reference value according to the age of the examinee by reading the age of the examinee associated with such an ID. It is needless to say that the examiner may operate the switch unit 6b and directly input the age of the examinee to set the reference value for each examinee. Further, the reference value for each examinee may be set by the examiner operating the switch unit 6b and selecting any reference value from a list.

In the present example, a configuration in which the correction power for correcting the subject eye E is gradually changed in the refractive correction examination with respective to the subject eye E, has been described as an example, but the present invention is not limited thereto. For example, a configuration may be adopted in which the correction power is changed from a predetermined correction power to a desired correction power each time in the refractive correction examination with respect to the subject eye E. In this case, the control means of the optometry system changes the control state of the correction means from the initial state to a first correction state, and returns to the initial state after the response in the first correction state is completed. Thereafter, the control means changes the control state of the correction means from the initial state to a second correction state different from the first correction state. That is, when changing the correction state by the correction means, the control means once changes the correction state to the initial state, and then changes the correction state to the next correction state.

For example, the initial state (initial value) of the correction power for correcting the subject eye E may be set based on the objective value of the examinee. For example, a correction power (for example, a value of +2.0D from the objective value) predicted from the objective value of the examinee, at which the adjustment of the subject eye E does not work may be set. It is needless to say that any value may be set. Further, for example, in a case where the correction power of the subject eye E is set to the initial value, the reaction time in a case where the correction power of the subject eye E is switched by predetermined steps (for example, 0.25D intervals) is sequentially acquired. At this time, in a case where the correction power of the subject eye E is switched to the next step, each time, the correction power is returned to the initial value.

As an example, in a case where the objective value of the subject eye E is -2.0D, the initial value of the correction power is set to 0D. The controller 70 switches the correction power of the subject eye E from 0D to -0.25D, and acquires the reaction time from the correction of the subject eye E to -0.25D to the time when a response is obtained. Subsequently, the controller 70 returns the correction power of the subject eye E from -0.25D to 0D, then switches the correction power of the subject eye E from 0D to -0.5D, and similarly acquires the reaction time in a case where the subject eye E is corrected by -0.5D. Subsequently, the controller 70 returns the correction power of the subject eye E from -0.5D to 0D, then switches the correction power of the subject eye E from 0D to -0.75D, and similarly acquires the reaction time in a case where the subject eye E is corrected by -0.75D.

Such switching of the correction power may be repeated until at least the correction power of the subject eye E reaches the objective value (-2.0D). For example, in a case of repeating the switching of the correction power even in a case where the correction power of the subject eye E exceeds the objective value, the repetition may be ended when no change is observed in the reaction time. It is needless to say that, even in a case where the refractive correction examination is performed in this manner, when an answer input from the examinee is not obtained, a voice guide for leading the examinee may be appropriately generated.

For example, the controller 70 may create graph data based on each reaction time obtained by repeatedly switching the correction power of the subject eye E, and display the graph data. For example, accordingly, it is possible to understand the transition of the reaction time reflecting the time required for adjustment of the subject eye E. As an example, since the change in the plotted points becomes small (or hardly changes) at the correction power at which the adjustment of the subject eye E works, the correct eye refractive power of the subject eye E can be estimated.

In the present example, a configuration in which the reaction times at all different start timings obtained during the refractive correction examination with respect to the subject eye E are displayed as graph data in a comparable manner, has been described as an example, but the present invention is not limited thereto. In the present example, the reaction times at at least two different start timings obtained during the refractive correction examination may be displayed in a comparable manner. For example, in this case, the assistant A or the examiner D may select any of the plurality of correction powers, and the controller 70 may display the corresponding reaction time according to the selection signal. In addition, for example, in this case, the reaction time at any correction power may be displayed together with the graph data described above.

In the present example, a configuration in which the refractive correction examination of the subject eye E is performed has been described as an example, but the present invention is not limited thereto. It is needless to say that, in the present example, at least one of the naked eye visual acuity examination, the red-green examination, the cross-cylinder examination, or the like may be performed on the subject eye E. In these cases, the controller 70 can obtain the reaction time of the examinee based on the start timing at which any one of the display 31, the light projecting optical system 30, and the correction optical system 60 is controlled to switch the visual target or the correction power, and the response timing at which the examinee responds with respect to this switching.

As an example, in the naked eye visual acuity examination of the subject eye E, the visual acuity value of the visual target presented to the subject eye E is switched in consideration of whether the response of the subject eye E is correct or incorrect. Therefore, the controller 70 may store the timing (start timing) at which the visual acuity value of the visual target is switched by controlling the display 31 and the response timing at which the response of the examinee is obtained.

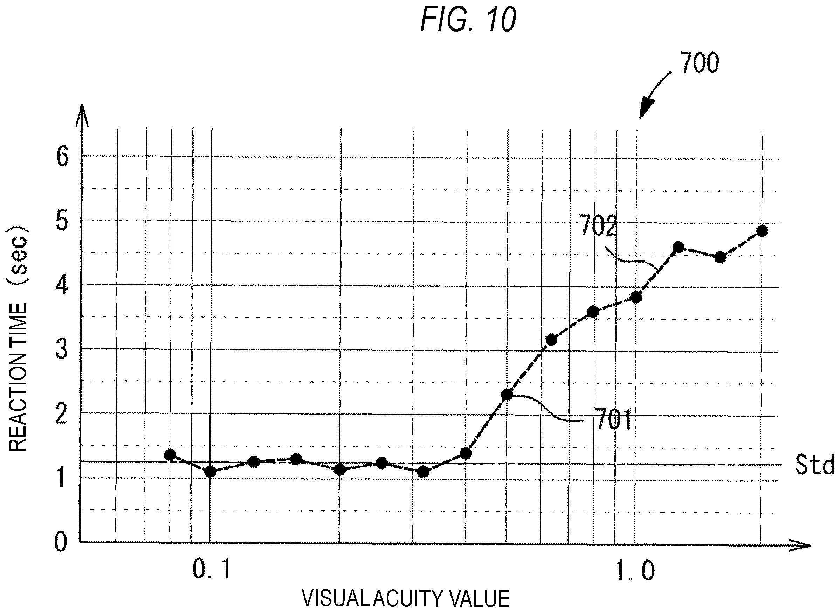

FIG. 10 is an example of a graph 700 showing a relationship between a reaction time and a visual acuity value in a naked eye visual acuity examination. For example, the vertical axis of the graph 700 is the reaction time of the examinee, and the horizontal axis is the visual acuity value of the visual target presented to the subject eye E. For example, the graph 700 may include a plurality of plotted points 701 at which the reaction time of the examinee is plotted for each visual acuity value, a broken line 702 generated based on the plurality of plotted points 701, the reference line Std generated based on the reference value of the reaction time of the examinee, and the like. For example, in a case of performing a naked eye visual acuity examination for the subject eye E, the visual acuity value of the visual target to be presented is normally changed step by step. However, the examiner may change the visual acuity value, for which the reaction time of the examinee has shortened, by two steps, based on the transition of the plotted points 701.

In the red-green examination of the subject eye E, the spherical power for correcting the subject eye E is switched in consideration of the correctness of the response of the subject eye E. Therefore, the controller 70 may store the timing (start timing) at which the spherical power is switched by controlling the light projecting optical system 30 and the response timing at which the response of the examinee is obtained. Further, the controller 70 may generate a graph in which the vertical axis is the reaction time of the examinee and the horizontal axis is the spherical power for correcting the subject eye E.

In addition, in the cross-cylinder examination of the subject eye E, the astigmatism axis angle for correcting the subject eye E is switched in consideration of the correctness of the response of the subject eye E. Therefore, the controller 70 may store the timing (start timing) at which the astigmatism axis power is switched by controlling the correction optical system 60 and the response timing at which the response of the examinee is obtained. Further, the controller 70 may generate a graph in which the vertical axis is the reaction time of the examinee and the horizontal axis is the astigmatism axis angle for correcting the subject eye E.

In the present example, a configuration in which, in the refractive correction examination with respect to the subject eye E, the spherical power for correcting the subject eye E is switched, and the graph 600 showing the relationship between the reaction time of the examinee and the spherical power is displayed, has been described as an example, but the present invention is not limited thereto. For example, a configuration may be adopted in which, in the refractive correction examination with respect to the subject eye E, the cylindrical power of the subject eye E is switched, and a graph showing the relationship between the reaction time of the examinee and the cylindrical power is displayed. Further, for example, a configuration may be adopted in which, in the refractive correction examination with respect to the subject eye E, the astigmatism axis angle of the subject eye E is switched, and a graph showing the relationship between the reaction time of the examinee and the astigmatism axis angle is displayed. In a case where all of the spherical power, the cylindrical power, and the astigmatism axis angle for correcting the subject eye E are switched, respective graphs may be generated and displayed.

In the present example, a configuration in which, in the refractive correction examination with respect to the subject eye E, only the correction power for correcting the subject eye E is changed without changing the visual acuity value of the visual target presented to the subject eye E, has been described as an example, but the present invention is not limited thereto. It is needless to say that, in the refractive correction examination with respect to the subject eye E, both the visual acuity value of the visual target presented to the subject eye E and the correction power for correcting the subject eye E may be changed. In this case, a graph showing the relationship between the reaction time and the visual acuity value of the examinee and a graph showing the relationship between the reaction time of the examinee and each correction power may be generated. Further, in this case, a graph that threedimensionally represents the relationship of the reaction time, the visual acuity value of the examinee, and each correction power may be generated.

In the present example, a configuration in which the examiner determines the next operation based on the reaction time of the subject eye E has been described as an example, but the present invention is not limited thereto. For example, in the present example, the controller 70 may automatically determine the next operation, based on the reaction time of the subject eye E. For example, the controller 70 may set a predetermined allowable range with respect to the reference value of the reaction time of the examinee, and may change the visual acuity value of the visual target to be switched next, based on whether or not the reaction time of the examinee exceeds the allowable range. More specifically, in a case where the reaction time of the examinee is approximately the same as the reference value (that is, within the allowable range), the visual acuity value of the visual target may be switched to a value one step higher. In addition, in a case where the reaction time of the examinee is significantly different from the reference value (that is, in a case where the reaction time is out of the allowable range), the visual acuity value of the visual target may be switched to a value two steps higher.

Further, for example, the controller 70 may set a predetermined allowable range with respect to the reference value of the reaction time of the examinee, and may change the correction power to be switched next, based on whether or not the reaction time of the examinee exceeds the allowable range. More specifically, in a case where the reaction time of the examinee is approximately the same as the reference value (that is, within the allowable range), the correction power may be switched by a small step (for example, 0.25D intervals). Further, in a case where the reaction time of the examinee is significantly different from the reference value (that is, in a case where the reaction time is out of the allowable range), the correction power may be switched by large steps (for example, 0.5D intervals).

For example, in this manner, the optometry system according to the present example can control the operation of the optometry system based on the reaction time of the examinee. The optometry system can determine the suitability of the state of the optometry system using the reaction time of the examinee, and control the operation of the optometry system based on the result. As a result, the visual acuity value of the visual target to be presented to the subject eye next and the correction power for correcting the subject eye next are appropriately selected, thereby optimizing the contents of the examination and shortening the examination time.

In the present example, a configuration in which the reaction time of the examinee is displayed on the monitor 6a has been described as an example, but the present invention is not limited thereto. In the present example, in addition to the reaction time of the examinee, information obtained based on the reaction time of the examinee may be displayed on the monitor 6a. As an example, in a case where there is a predetermined difference in reaction time between the left eye and the right eye in a case of being corrected with each correction power, the predetermined difference may be displayed, or a message indicating that there is a predetermined difference may be displayed. The predetermined difference may be preset from experiments, simulations, and the like. For example, with such a configuration, the examiner can easily estimate the cause of the large difference in reaction time between the left eye and the right eye (a possibility of a disease in the subject eye E, a possibility that the correction power for correcting the left eye and the right eye is misaligned, or the like).

### REFERENCE SIGNS LIST

7 Measurement unit
10 Objective measurement optical system
25 Subjective measurement optical system
70 Controller
75 Memory
81 Deflection mirror
84 Reflection mirror
85 Concave mirror
100 Subjective optometry device

## Claims

1. An optometry system for subjectively measuring an optical characteristic of a subject eye by presenting an examination visual target to the subject eye, the optometry system comprising:
a setting means that sets a reference value based on a reaction time of an examinee with respect to the examination visual target;
a response input means by which the examinee reading the examination visual target inputs an answer;
a control means that automatically proceeds with an examination, based on an input signal from the response input means; and
a leading information output means that outputs leading information, during the examination of the subject eye, for leading the examinee to input the answer, based on the reference value set by the setting means.

2. The optometry system according to claim 1,
wherein the leading information output means repeatedly outputs the leading information, in a case where no signal from the response input means is obtained after a predetermined time set for repeating the leading information has elapsed.

3. The optometry system according to claim 2,
wherein the leading information output means changes the leading information from first leading information to second leading information that is at least partially different from the first leading information, to output the leading information, according to the number of times of output of the leading information.

4. The optometry system according to any one of claims 1 to 3,
wherein the optometry includes a plurality of examination items, and
the setting means sets the reference value according to the examination items.

5. The optometry system according to any one of claims 1 to 4, further comprising:
a reaction time acquisition means that acquires the reaction time, based on a start timing at which measurement of the subject eye is started and a response timing at which the examinee responds with respect to the examination visual target,
wherein the setting means sets the reference value, based on the reaction time acquired by the reaction time acquisition means.

6. The optometry system according to claim 5,
wherein the reaction time acquisition means acquires the reaction time in real time, during the examination,
the setting means changes the reference value from a first reference value to a second reference value different from the first reference value to set the reference value, based on the reaction time acquired in real time by the reaction time acquisition means, and
the leading information output means outputs the leading information, based on the second reference value.

7. The optometry system according to claim 5 or 6, further comprising:
a visual target presenting means that emits a target light flux toward the subject eye to present the examination visual target to the subject eye,
wherein the control means controls the visual target presenting means to automatically proceed with the examination, and
the reaction time acquisition means:
acquires a timing at which the control means controls the visual target presenting means, as the start timing; and
acquires a timing at which the input signal from the response input means is acquired, as the response timing.

8. The optometry system according to any one of claims 5 to 7, further comprising:
a correction means that changes an optical characteristic of the target light flux to change a correction power for correcting the subject eye,
wherein the control means controls the correction means to automatically proceed with the examination, and
the reaction time acquisition means acquires a timing at which the control means controls the correction means, as the first timing.

9. The optometry system according to claim 1, further comprising:
a visual target presenting means that emits a target light flux toward the subject eye;
a correction means that changes an optical characteristic of the target light flux;
a control means that controls an operation of the optometry system; and
an output means that outputs the reaction time,
wherein the reaction time is a reaction time based on a start timing at which measurement of the subject eye is started, which is a start timing at which the visual target presenting means or the correction means is controlled by the control means, and a response timing after the start timing, which is a response timing at which a response signal for the measurement is input by the response input means, and is a reaction time required from start of the measurement to response.

10. An optometry system for subjectively measuring an optical characteristic of a subject eye, the optometry system comprising:
a visual target presenting means that emits a target light flux toward the subject eye;
a correction means that changes an optical characteristic of the target light flux;
a control means that controls an operation of the optometry system;
a reaction time acquisition means that acquires a reaction time required from start of measurement to a response, based on a start timing at which the measurement of the subject eye is started, which is a start timing at which the visual target presenting means or the correction means is controlled by the control means, and a response timing after the start timing, which is a response timing at which a response signal for the measurement is input by the response input means; and
an output means that outputs the reaction time acquired by the reaction time acquisition means.

11. The optometry system according to claim 10,
wherein the reaction time acquisition means acquires a first reaction time and a second reaction time, that are acquired at different timings, as the reaction time,
at least one of the start timing or the response timing of the first reaction time and the second reaction time is different, and
the output means outputs the first reaction time and the second reaction time in a comparable manner.

12. The optometry system according to claim 10 or 11,
wherein the control means is capable of controlling at least one of the visual target presenting means or the correction means, and controls an operation of the optometry system based on the reaction time acquired by the reaction time acquisition means.

13. The optometry system according to claim 12, further comprising:
a state acquisition means that acquires a state of the optometry system in a case where the visual target presenting means or the correction means is controlled by the control means; and
a determination means that determines suitability of a state of the optometry system acquired by the state acquisition means, based on the reaction time acquired by the reaction time acquisition means,
wherein the control means controls an operation of the optometry system, based on a determination result of the determination means.

14. The optometry system according to any one of claims 10 to 13,
wherein the output means outputs a state of the optometry system acquired by the state acquisition means, together with the reaction time acquired by the reaction time acquisition means.

15. The optometry system according to claim 14,
wherein the output means outputs data indicating a relationship between the reaction time and a state of the optometry system.

16. The optometry system according to claim 15,
wherein the output means outputs graph data indicating a relationship between the reaction time and a state of the optometry system.

17. The optometry system according to claim 16,
wherein the output means outputs graph data indicating a relationship between the reaction time and a correction power set by the correction means.

18. An optometry program used in an optometry system for subjectively measuring an optical characteristic of a subject eye by presenting an examination visual target to the subject eye, the optometry program being executed by a processor to cause the optometry system to perform:
a setting step of setting a reference value based on a reaction time of an examinee with respect to the examination visual target;
an answering step in which the examinee reading the examination visual target inputs an answer;
a control step of automatically proceeding with an examination, based on an input signal from the response input means; and
a leading information output step of outputting leading information, during the examination of the subject eye, for leading the examinee to input the answer, based on the reference value set by the setting means.

19. An optometry program used in an optometry system for subjectively measuring an optical characteristic of a subject eye, the optometry system including a visual target presenting means that emits a target light flux toward the subject eye, a correction means that changes an optical characteristic of the target light flux, and a control means that controls an operation of the optometry system, the control means being capable of controlling at least one of the visual target presenting means or the correcting means, the optometry program being executed by the control means to cause the optometry system to perform:
a reaction time acquisition step of acquiring a reaction time required from start of measurement to response based on a start timing at which the measurement of the subject eye is started, which is a start timing at which the visual target presenting means or the correction means is controlled by the control means, and a response timing after the start of the measurement, which is a response timing at which a response signal for the measurement is input by response input means, and
an output step of outputting the reaction time acquired by the reaction time acquisition means.
